# EUROPEAN PATENT APPLICATION

(11) **EP 2 982 765 A1**
(43) Date of publication of application: **10.02.2016**
(21) Application number: 14180306.4
(22) Date of filing: 08.08.2014
(51) Int. Cl.: C12Q 1/68

(54) **Glycodelin as diagnostic and prognostic marker and for monitoring treatment of lung diseases**

(71) Applicant: Thoraxklinik Heidelberg GmbH, 69126 Heidelberg (DE)
(72) Inventor: Schneider, Marc, 65462 Ginsheim-Gustavsburg (DE); Meister, Michael, 68309 Mannheim (DE); Muley, Thomas, 69509 Mörlenbach (DE)
(74) Representative: Grahn, Sibylla Maria

(57) **Abstract**

The present invention relates to glycodelin for use in the diagnosis, prognosis and/or treatment monitoring of a lung disease. The present invention further relates to a method for the diagnosis, prognosis and/or treatment monitoring of a lung disease, comprising the step of determining the presence of glycodelin in serum, plasma and/or tissue.

The present invention further relates to glycodelin for use in modulating the immune system in subjects with lung transplantation, preferably in order to prevent organ recipient from allograft rejection, comprising treatment of the lung with glycodelin before, during and/or after said lung transplantation and a respective method.

## Description

The present invention relates to glycodelin for use in the diagnosis, prognosis and/or treatment monitoring of a lung disease. The present invention further relates to a method for the diagnosis, prognosis and/or treatment monitoring of a lung disease, comprising the step of determining the presence of glycodelin in serum, plasma and/or tissue.

The present invention further relates to glycodelin for use in modulating the immune system in subjects with lung transplantation, preferably in order to prevent organ recipient from allograft rejection, comprising treatment of the lung with glycodelin before, during and/or after said lung transplantation and a respective method.

### BACKGROUND OF THE INVENTION

With about 1.3 million cases worldwide, lung cancer is the leading cause for cancer-related death. Based on histo-pathological characterization, lung cancer can be divided in two subpopulations: non-small cell lung cancer (NSCLC), which accounts for more than 80 % of all cases, and small-cell lung cancer (SCLC) (Giaccone et al., 2004). In recent years chemotherapeutic treatments of lung cancer patients are progressively supplemented with precision therapy based on genetic analyses due to the still very low 5-year survival rate in NSCLC (Rosell et al., 2013). Sequencing of lung carcinoma demonstrated a high somatic mutation rate of *TP53, KRAS* and *EGFR* gene (Govindan et al., 2012). First- and second-line drugs like gefitinib and erlotinib that target the epidermal growth factor receptor can promote progression-free survival (Rosell et al., 2012).

In general, altered cells present markers on cell surface and secret chemokines to attract lymphocytes such as natural killer cells or cytotoxic T cells. Tumor microenvironment is strongly defined by extracellular matrix reorganization and chemokine secretion of tumor that lead to a barrier for lymphocytes (Salmon et al., 2012). Infiltration of NSCLCs with lymphocytes is associated with better disease-free survival (Al-Shibli et al., 2008). Thus, immune therapy has an adequate potentiality to stimulate patient's immune system response and attack cancer more effectively. Manipulation of NK cells and Natural Killer T cells (iNKT) seems to be a good tool for cancer immunotherapy (Terme et al., 2008; Motohashi et al., 2006).

There is a need in the art for improved means and methods for diagnosing lung diseases and for therapy monitoring.

### SUMMARY OF THE INVENTION

According to the present invention this object is solved by providing glycodelin for use in the diagnosis, prognosis and/or treatment monitoring of a lung disease,
wherein the lung disease is selected from thoracic malignancies, mesothelioma, lung metastasis from distant malignant diseases and benign thoracic diseases.

According to the present invention this object is solved by providing glycodelin for use in modulating the immune system in a subject with lung transplantation,
comprising treatment of the lung with glycodelin before, during and/or after said lung transplantation.

According to the present invention this object is solved by a method for the diagnosis, prognosis and/or treatment monitoring of a lung disease, comprising the step of
determining the presence of glycodelin in serum, plasma and/or tissue, wherein the lung disease is selected from thoracic malignancies, mesothelioma, lung metastasis from distant malignant diseases and benign thoracic diseases.

According to the present invention this object is solved by a method for modulating the immune system in a subject with lung transplantation, comprising the step of treatment of the lung with glycodelin before, during and/or after said lung transplantation.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1.0 and 1.5 ng/ml" should be interpreted to include not only the explicitly recited values of 0.8 to 1.5, but also include individual values and sub-ranges within the indicated range. Thus, included in this numerical range are individual values such as 1.0, 1.05, 1.1... 1.35, 1.4, 1.45, 1.5 and sub-ranges such as from 1.05 to 1.2, 1.0 to 1.2, 1.05 to 1.3, etc. This same principle applies to ranges reciting only one numerical value, such as "about 1.1 ng/ml". Furthermore, such an interpretation should apply regardless of the breadth of the range or the characteristics being described.

### Biomarker glycodelin

As discussed above, the present invention provides glycodelin for use in the diagnosis, prognosis and/or treatment monitoring of a lung disease.

Glycodelin, initially described as placental protein 14 (PP14) or progesterone-associated endometrial protein (*PAEP*), is strongly associated with trophoblastic invasiveness (Lam et al., 2009). Down regulation of glycodelin leads to an increased activation of maternal immune system and can result in abortion during the first trimester. Glycodelin is differentially glycosylated depending on gender and function (Morris et al., 1996). Glycodelin A, the immunosuppressive form of glycodelin, functions as proliferation suppressor and apoptosis inducer of T cells, monocytes, B cells and NK cells (Alok et al., 2009). Atypical glycodelin expression is found in hormone-related breast cancer, endometrial cancer and ovarian cancer (Seppala et al., 2009). Recent studies demonstrated a glycodelin expression in melanoma cells (Ren et al., 2010). In the present invention, we show that glycodelin is highly expressed in lung cancer tissue. Glycodelin reorganizes tumor cell expression profile to undergo immune system defense and is a highly suitable biomarker to track tumor progression, recurrence or metastatic spread.

*PAEP* is the gene that encodes the glycodelin protein.

Genbank: Gene ID: 5047

Accession number: NC_000009.12

Nucleotide sequence: SEQ ID NO. 1

Glycodelin

Genbank: Gene ID: 5047

Accession number: NP_001018059.1

Amino acid sequence: SEQ ID NO. 2

Glycodelin when used herein also refers to / comprises glycodelin A.

According to the invention, the lung disease is preferably selected from:
- thoracic malignancies
   such as lung cancer,
- mesothelioma,
- lung metastasis from distant malignant diseases (such as colon cancer, renal cell carcinoma, extrathoracic sarcomas),
   and
- benign thoracic diseases,
   such as asthma, pulmonary hypertension, COPD, Interstitial Lung disease (ILD).

Preferably, lung cancer is selected from
- non-small cell lung cancer (NSCLC)
   such as adenocarcinoma, squamous cell carcinoma, large-cell lung carcinoma
- small cell lung cancer (SCLC);
- lung carcinoid.

In one embodiment, the use according to the invention comprises determining the presence of glycodelin in serum, plasma and/or tissue, such as tumor tissue.

Preferably, determining the presence of glycodelin comprises
- determining presence / expression of glycodelin mRNA,
   preferably via quantitative detection of the mRNA (such as qPCR),
- determining presence / expression of glycodelin
   preferably via immune assay(s) (such as via an ELISA) or imununohistochemistry (such as of tissue sections), such as by using a specific anti-glycodelin antibody.

Preferably, in serum or plasma or tissue glycodelin level is determined; in tissue glycodelin mRNA and protein expression is determined.

Preferably, glycodelin is not detectable in normal lung parenchyma.

Preferably, glycodelin is detectable in bronchial epithelium of the lung and in tumor tissue. More preferably glycodelin is increased in bronchial epithelium of the lung and in tumor tissue compared to normal lung parenchyma or glycodelin is above a threshold (value) or cut-off (value).

In one embodiment, a threshold (value) or cut-off (value) for glycodelin serum level is established in a control group of patients or subjects. The control group of patients are preferably patients/subjects with benign lung diseases, extrapulmonary benign diseases (i.e. cardiovascular diseases, etc.) or are healthy (male and female each preferably n = 50).

For example, a threshold (value) or cut-off (value) can be determined by Receiver Operating Characteristics (ROC) analyses.

For example, a threshold (value) or cut-off (value) for ELISA can be determined by Receiver Operating Characteristics (ROC) analyses.

For example, in the preliminary cohort, two cut-off values can be determined:
1. Optimized relation between specificity and sensitivity is found at about 1.1 ng/ml (within a range of about 1.0 and 1.5 ng/ml).
2. At 95% specificity (frequently used as a specification of immunotests) a cut-off value of about 3.5 ng/ml glycodelin (within a range of about 3 and 4 ng/ml) is found.

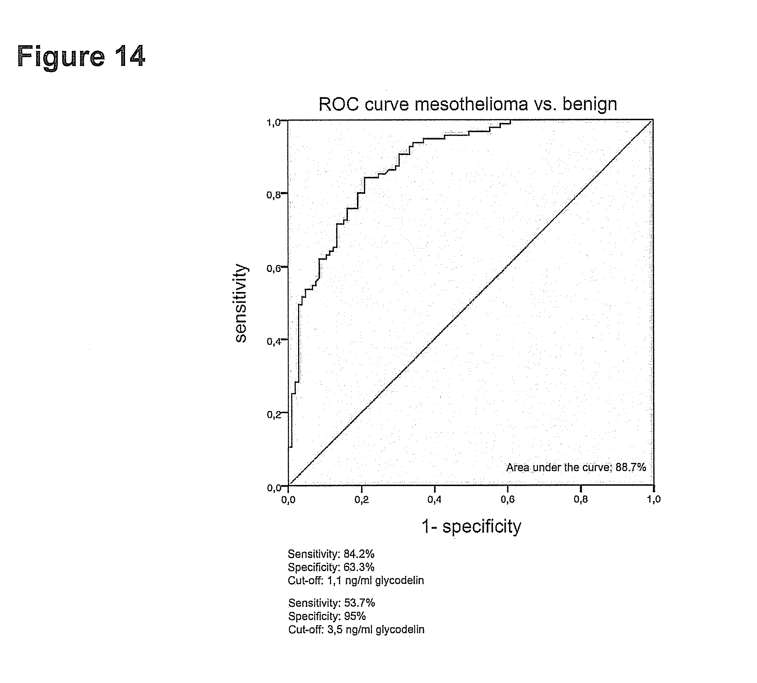

For example, for the differentiation between mesothelioma and benign lung diseases a ROC curve is shown in Fig. 14 with an area under the curve (AUC) of about 88.7% (within a range of about 84 and 93 %, such as 84.3 to 93.1 %).

For glycodelin mRNA detection, analyses comparing mRNA in tumor and normal tissues are preferred.

For detection of the expression of glycodelin (preferably via immunohistochemistry using antibodies), a scoring system (i.e. H-Score) can be applied.

In one embodiment, female patients or subjects are preferably in postmenopause state, in order to exclude increased glycodelin serum/plasma levels found during menstruation cycle.

In one embodiment, diagnosis of a lung disease comprises the early diagnosis of the lung disease.

Preferably, increased glycodelin levels in serum/plasma and/or tissue indicate a lung disease, such as a malignant thoracic disease (primary or metastatic).

In one embodiment, prognosis of a lung disease comprises:
- assessment of progression-free survival,
- assessment of disease-free survival
   and/or
- assessment of overall survival,- assessment of survival.

Preferably, poorer survival is prognosed when glycodelin levels in serum/plasma are increased and/or when glycodelin mRNA and protein expression in tissue is increased.

In one embodiment, "treatment monitoring" comprises determining the presence of glycodelin over time / over a period of time,
such as
before, during and/or after therapy
such as surgery, chemo-, immune- and/or radiotherapy; or combined modality treatment,
and/or
during follow-up after therapy.

Treatment monitoring during follow-up therapy is carried out, for example, in order to detect recurrence or metastatic spread of malignant thoracic diseases.

Preferably, a therapy (such as surgery, chemo-, immune- and/or radiotherapy or combined modality treatment) is found successful, if the glycodelin level (glycodelin serum/plasma level and/or mRNA tissue level) during and/or after therapy is low or decreased compared to the glycodelin level before said therapy / pre-therapeutic values or levels.

For example, an increase in glycodelin serum/plasma and/or tissue levels or mRNA expression can indicate the necessity for modifications of a patient's/subject's therapeutic schedule/regimen (such as starting immune-modulating therapies; change of drug).

### Diagnosis, prognosis and monitoring methods

As discussed above, the present invention provides a method for the diagnosis, prognosis and/or treatment monitoring of a lung disease.

Said method comprises the step of
determining the presence of glycodelin in serum, plasma and/or tissue, such as tumor tissue.

As discussed above, the lung disease is preferably selected from:
- thoracic malignancies
   such as lung cancer,
- mesothelioma,
- lung metastasis from distant malignant diseases (such as colon cancer, renal cell carcinoma, extrathoracic sarcomas),
   and
- benign thoracic diseases,
   such as asthma, pulmonary hypertension, COPD, Interstitial Lung disease (ILD).

As discussed above, lung cancer is preferably selected from
- non-small cell lung cancer (NSCLC)
   such as adenocarcinoma, squamous cell carcinoma, large-cell lung carcinoma
- small cell lung cancer (SCLC);
- lung carcinoid.

In one embodiment, the use according to the invention comprises determining the presence of glycodelin in serum, plasma and/or tissue, such as tumor tissue..

Preferably (and as discussed above), determining the presence of glycodelin comprises
- determining presence / expression of glycodelin mRNA,
   preferably via quantitative detection of the mRNA
   (such as qPCR),
- determining presence / expression of glycodelin
   preferably via immune assay(s) (such as via an ELISA) or immunohistochemistry (such as of tissue sections), such as by using a specific anti-glycodelin antibody.

Preferably, in serum or plasma glycodelin level is determined; in tissue glycodelin mRNA and protein expression is determined.

Preferably, glycodelin is not detectable in normal lung parenchyma.

Preferably, glycodelin is detectable in bronchial epithelium of the lung and in tumor tissue. More preferably glycodelin is increased in bronchial epithelium of the lung and in tumor tissue compared to normal lung parenchyma or glycodelin is above a threshold (value) or cut-off (value)

In one embodiment, a threshold (value) or cut-off (value) for glycodelin serum level is established in a control group of patients or subjects. The control group of patients are preferably patients/subjects with benign lung diseases, extrapulmonary benign diseases (i.e. cardiovascular diseases, etc.) or are healthy (male and female each preferably n = 50).

For example, a threshold (value) or cut-off (value) can be determined by Receiver Operating Characteristics (ROC) analyses.

As discussed above, for example, a threshold (value) or cut-off (value) for ELISA can be determined by Receiver Operating Characteristics (ROC) analyses.

For example, in the preliminary cohort, two cut-off values can be determined:
1. Optimized relation between specificity and sensitivity is found at about 1.1 ng/ml (within a range of about 1.0 and 1.5 ng/ml).
2. At 95% specificity (frequently used as a specification of immunotests) a cut-off value of about 3.5 ng/ml glycodelin (within a range of about 3 and 4 ng/ml) is found.

For example, for the differentiation between mesothelioma and benign lung diseases a ROC curve is shown in Fig. 14 with an area under the curve (AUC) of about 88.7% (within a range of about 84 and 93 %, such as 84.3 to 93.1 %).

For glycodelin mRNA detection, analyses comparing mRNA in tumor and normal tissues are preferred.

For detection of the expression of glycodelin (preferably via immunohistochemistry using antibodies), a scoring system (i.e. H-Score) can be applied.

In one embodiment, female patients or subjects are preferably in postmenopause state, in order to exclude increased glycodelin serum/plasma levels found during menstruation cycle.

In one embodiment, diagnosis of a lung disease comprises the early diagnosis of the lung disease.

Preferably, increased glycodelin levels in serum/plasma and/or tissue indicate a lung disease, such as a malignant thoracic disease (primary or metastatic).

In one embodiment, prognosis of a lung disease comprises:
- assessment of progression-free survival,
- assessment of disease-free survival
   and/or
- assessment of overall survival,- assessment of survival.

Preferably, poorer survival is prognosed when glycodelin levels in serum/plasma are increased and/or when glycodelin mRNA and protein expression in tissue is increased.

In one embodiment, treatment monitoring comprises determining the presence of glycodelin over time / over a period of time,
such as
before, during and/or after therapy
such as surgery, chemo-, immune- and/or radiotherapy; or combined modality treatment,
and/or
during follow-up after therapy.

Treatment monitoring during follow-up therapy is carried out, for example, in order to detect recurrence or metastatic spread of malignant thoracic diseases.

Preferably, a therapy (such as surgery, chemo-, immune- and/or radiotherapy or combined modality treatment) is found successful, if the glycodelin level (glycodelin serum/plasma level and/or mRNA tissue level) during and/or after therapy is low or decreased compared to the glycodelin level before said therapy / pre-therapeutic values or levels.

For example, an increase in glycodelin serum/plasma and/or tissue levels or mRNA expression can indicate the necessity for modifications of a patient's/subject's therapeutic schedule/regimen (such as starting immune-modulating therapies; change of drug).

*Immunomodulation or immunosuppression before, during and*/*or after lung transplantation,* As discussed above, the present invention provides glycodelin for use in modulating the immune system in a subject / patient with lung transplantation,

Said use comprises:
treatment of the lung with glycodelin before, during and/or after said lung transplantation.

Preferably, modulating the immune system in a subject comprises supressing the immune system.

The treatment before lung transplantation can be carried out by flushing the lung with a glycodelin solution, inhaled vaporised glycodelin or *ex vivo* gene delivery.

Said treatment preferably prevents organ recipient from allograft rejection.

In one embodiment, said use comprises an additional treatment of patients or subjects with vaporised glycodelin after transplantation. Said additional treatment preferably increases the acceptance of organ.

As discussed above, the present invention provides a method for modulating the immune system in a subject / patient with lung transplantation.

Said method comprises the step of
treatment of the lung with glycodelin before, during and/or after said lung transplantation.

Preferably, modulating the immune system in a subject comprises supressing the immune system.

The treatment before lung transplantation is preferably carried out by flushing the lung with a glycodelin solution, inhaled vaporised glycodelin or *ex vivo* gene delivery.

Said treatment preferably prevents organ recipient from allograft rejection.

In one embodiment, said method comprises an additional treatment of patients or subjects with vaporised glycodelin after transplantation. Said additional treatment preferably increases the acceptance of organ.

### Preferred embodiments

In recent years, several new immune therapy strategies for non-small cell lung cancer (NSCLC) patients have been developed. Nevertheless, the crosstalk between tumor and immune cells is poorly understood. Glycodelin (*PAEP*), described initially in pregnancy and trophoblastic implantation, is a secreted immunosuppressive glycoprotein. The present invention describes the expression and the role of glycodelin in lung diseases, in particular NSCLC and others, such as mesotheliom.

Glycodelin mRNA expression is elevated in more than 80% of all tumors (n=336) compared to matched normal tissue. Overall survival was found to be significantly reduced in women expressing high glycodelin mRNA-levels. Immunohistochemical staining of tumors showed inhomogeneous glycodelin protein expression. Glycodelin was detected in serum of patients expressing PAEP in tumors and levels correlated with recurrence and metastatic disease. Knockdown of PAEP with siRNAs in NSCLC cell lines resulted in up regulation of immune system modulatory factors like CXCL5, CXCL16, MICA/B, PDL1/2 and CD83 as well as proliferation stimulators EDN1 and HBEGF. A reorganization of cell structure was observed and led to a decreased migration of tumor cells. Treatment of cells with PMA, LPA and growth factors HBEGF and EGF induced glycodelin expression while siRNA knockdown of transcription factor MITF resulted in a strong decrease of expression. Altogether, the results presented here provide strong evidence for an immune modulatory function of glycodelin in NSCLC.

Furthermore, the present invention shows that glycodelin is a highly suitable biomarker to track tumor progression, recurrence or metastatic spread, but also to monitor treatment and therapy.

### - Discussion

Expression of glycodelin is significantly up regulated during implantation of trophoblast and supports its implantation by suppressing maternal immune system (Kao et al., 2002). Glycodelin A inhibits lymphocyte activity, proliferation and IL-2 secretion and increases apoptosis in T-cells (Alok et al., 2009; Soni et al., 2012. Up to now, the expression of glycodelin is well described mainly in hormone related cancer like breast cancer and ovarian cancer (Seppala et al., 2009). Recently, Zadran et al. found *PAEP* to be one of the most differentially regulated genes in a small cohort of lung adenocarcinoma compared to healthy patients (Zadran et al., 2013).

Here, we showed for the first time in a large cohort that glycodelin was highly expressed in non-small lung cancer. *PAEP* mRNA level in tumors was elevated in about 80% of all patients compared to normal tissue and up regulated in later tumor stages. A high *PAEP* expression was associated with shorter survival in squamous cell carcinoma. Especially women seemed to have a worse survival that was independent of tumor pathology but possibly influenced by lymph node status.

We demonstrated the expression of immune system modulatory glycodelin A. It might be possible that glycodelin expression and secretion within tumors is adapted to microenvironment to suppress activity of tumor infiltrating T cells and NK cells. In accordance to glycodelin staining in mice bronchus (Kunert-Keil et al., 2009) we found a strong expression of glycodelin in human bronchial epithelium. This observation confirmed the qPCR expression data of *PAEP.* We were not able to detect *PAEP* expression in about the half of all normal tissues and a very low expression level in general that could be explained by *PAEP* expression of bronchial epithelial cells. This contrasts with a study from 2011 (Kunert-Keil et al., 2011), where a polyclonal rabbit anti-glycodelin antibody was used and glycodelin was strongly stained in tumor adjacent normal tissue. Apparently, the antibody used was unspecific since our data demonstrated a very low mRNA expression of glycodelin in general and no detectable mRNA in about the half of all normal tissues. Moreover, a staining of normal lung alveolar cells with glycodelin antibody was negative. Specificity of the antibody used in the present invention was controlled by using *PAEP* specific siRNAs and blocking peptide.

We were able to detect high glycodelin concentrations in serum samples of lung cancer but not in healthy patients. Thus, our data clearly demonstrate, that a measurement of glycodelin in serum is useful for clinical follow up of patients that underwent excision of tumor, since we observed a correlation between tumor excision as well as recurrence and glycodelin serum level in most patients that primarily expressed glycodelin.

We observed the expression of glycodelin also in lymph nodes and metastasis. Our results suggest that glycodelin expression influences the spread of lung cancer. We showed that glycodelin was secreted by cell lines derived from breast cancer, melanoma and several lung cancer cell lines. Therefore, glycodelin is a suitable marker for cancer detection and monitoring, in particular of lung cancer.

Tumors often undergo an immunosurveillance through regulation of chemokine secretion and presentation of cell surface molecules. Immunoediting is important for tumors to hide from the immune system. In our work, we found several immune system modulators upregulated in the cell lines after loss of *PAEP.* MICA and MICB, both MHC-I related chains (MIC), are ligands for NKGD2, a receptor on the surface of NK cells and CD8⁺ T cells. For lung cancer, it has been reported that MICA and MICB were recognized by tumor infiltrating T cells (Groh et al., 1999). In contrast, the expression of immunosystem defensing factors *CD83, PDL-1* and *PDL-2* as well as chemokines *CXCL5* and *CXCL16* was strongly increased after knockdown of *PAEP.* PD-1 and its ligand PDL-1 are current targets for lung cancer immune therapy (Davies et al., 2014). CXCL5 and CXCL16 have been shown to be involved in angiogenesis and attracting CD4⁺ immune cells in NSCLC (Darash-Yahana et al., 2009). CD83 expression influenced CD4⁺ T cell development by stimulation of dendritic cells. We found that glycodelin knockout leads to a reorganization of immunosystem stimulating and silencing factors.

Our data furthermore indicate that glycodelin may also be involved in neovascularization trough an upregulation of prostaglandin-endoperoxide synthase 2 (*PTGS2*/*COX-2*) in cell lines after knockdown of *PAEP.* PTGS2 is described as a marker of angiogenesis. and promoted activity of regulatory T cells (Treg) (Sharma et al., 2005). Treg cells have been shown to infiltrate lung adenocarcinoma and to play an essential role in control of antitumor responses by blocking immune cells (Schneider et al., 2011).

As the knockdown of *PAEP* in our study especially regulated immune system relevant genes, we postulate that glycodelin expression and secretion is essential for lung cancer to escape from immune system.

In our study we showed that glycodelin expression is differentially regulated in lung cancer. PMA seemed to be a general inducer for *PAEP* that indicates a regulation via activation of protein kinase C. The knockdown of *PAEP* in cell lines resulted in an increased expression of several pathway regulating ligands. HBEGF and EGF led to an elevated expression and secretion of glycodelin in 2106T and MeWo cell lines, both derived from squamous cell carcinomas. Cell line H1975 contains an EGFR mutation resulting in a constitutive activation of EGFR signaling (Yu et al., 2007). This might be the reason why *PAEP* was not induced in this cell line. HBEGF was found to have different functions during pregnancy and provides cytoprotection of trophoblast cells during implantation (Jessmon et al., 2009).

In summary, we demonstrated the expression of glycodelin on mRNA and protein level in NSCLC. From our results and the known immunomodulating functions of glycodelin it is feasible to propose an immune system defense mechanism of lung tumors expressing and secreting glycodelin. Glycodelin produced by tumor cells might reduce the effect of current therapies using immune-checkpoint inhibitors (e.g. PDL1 targeted therapies), as it is able to remodel the host immune system and the tumor immune system defense. Our data strongly suggest that a detection of glycodelin in tumor and serum is helpful to design tumor specific immunotherapies for cancers, especially for NSCLC.

The following examples and drawings illustrate the present invention without, however, limiting the same thereto.

### BRIEF DESCRIPTION OF THE DRAWINGS

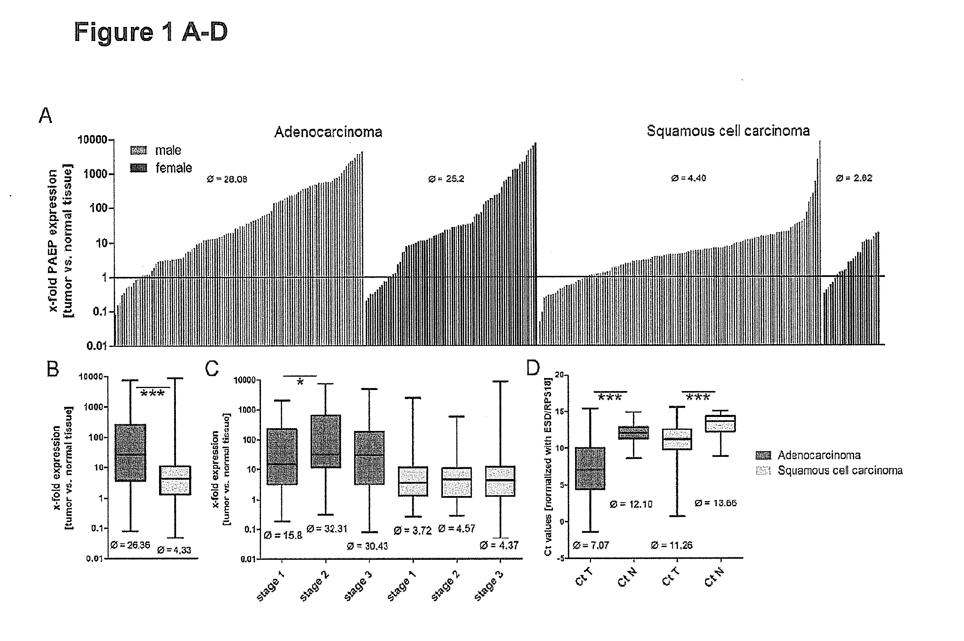

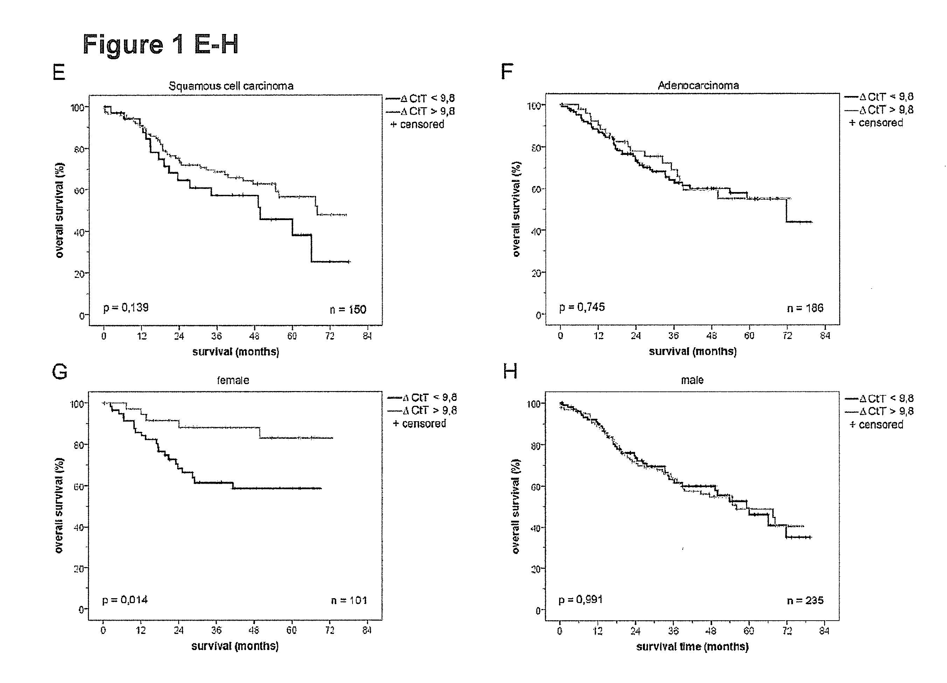

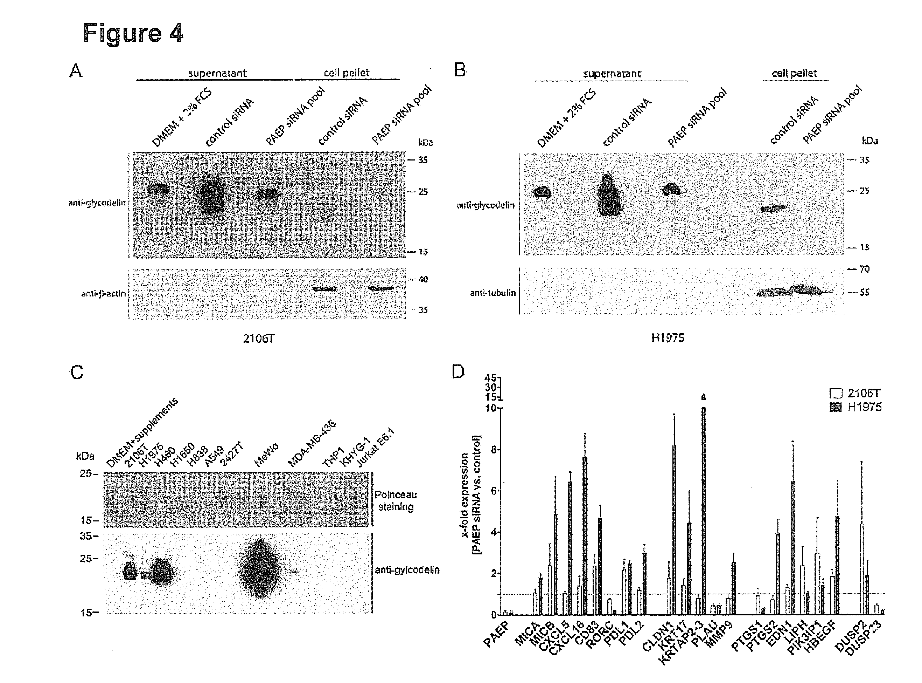

**Figure 1****.** *PAEP is highly overexpressed in lung cancel and leads to a decreased overall survival.*
   **A,** *PAEP*-expression in 337 lung tumor and corresponding normal tissues was quantified by qPCR and tumor vs. normal tissue expression.
   **B**-**D**, normalized tumor Ct values were visualized with GraphPad Prism.
   **E-H,** percentage overall survival is depicted for tumors with normalized Ct values <9.8 or >9.8.
   T = tumor, N = normal, *p<0,05, *** p<0,0001
**Figure 2****.** *Glycodelin as well as glycodelin A is strongly expressed in lung tumor tissue.* **A and B,** representative immunohistological stainings of lung tumor and normal tissues of glycodelin and glycodelin A are shown. A, CD8 was additionally stained to detect inflammation. **B,** a glycodelin A specific antibody was used to detect the immunomodulatory, glycosylated form of glycodelin.
**Figure 3****.** *Glycodelin is secreted from tumors*.
   A - D, glycodelin concentration was quantified by ELISA.
   **A,** patients were grouped related to the qPCR expression level of *PAEP* and serum level of glycodelin was determined before surgery.
   **B,** 10 randomly selected adenocarcinoma and corresponding normal lung tissue pieces were homogenized and glycodelin concentrations were measured.
   **C,** serum level was measured before and after excision of tumor.
   **D,** glycodelin concentration in serum was quantified before surgery and during follow up ward rounds. Patient 3 showed a recurrence of primary tumor. An adrenal gland metastasis of Patient 4 was resected after ward round 4.
   **E,** primary tumor and metastasis of patient 4 was stained for glycodelin.
   T = tumor, N = normal, R = recurrence, M = metastasis, WR = ward round, + = tumor dependent death
**Figure 4****.** *Glycodelin is expressed in NSCLC cell lines and modulates immunosurveillance.*
   **A and B,** indicated cell lines were transfected with a pool of four different *PAEP*-specific siRNAs for 72h. Medium was changed and 24 h later, supernatants as well as cells were collected and glycodelin. was detected by western blot. B-actin and tubulin were used as loading controls.
   **C,** an 80% confluent 12-well with the indicated cell lines each was cultivated with DMEM + supplements for 24 hours. Supernatants were collected and taken for western blot analyses of glycodelin. Poinceau-staining was used as a loading control.
   **D,** after 72 h knock-out *of PAEP,* expression of indicated genes was determined by qPCR.

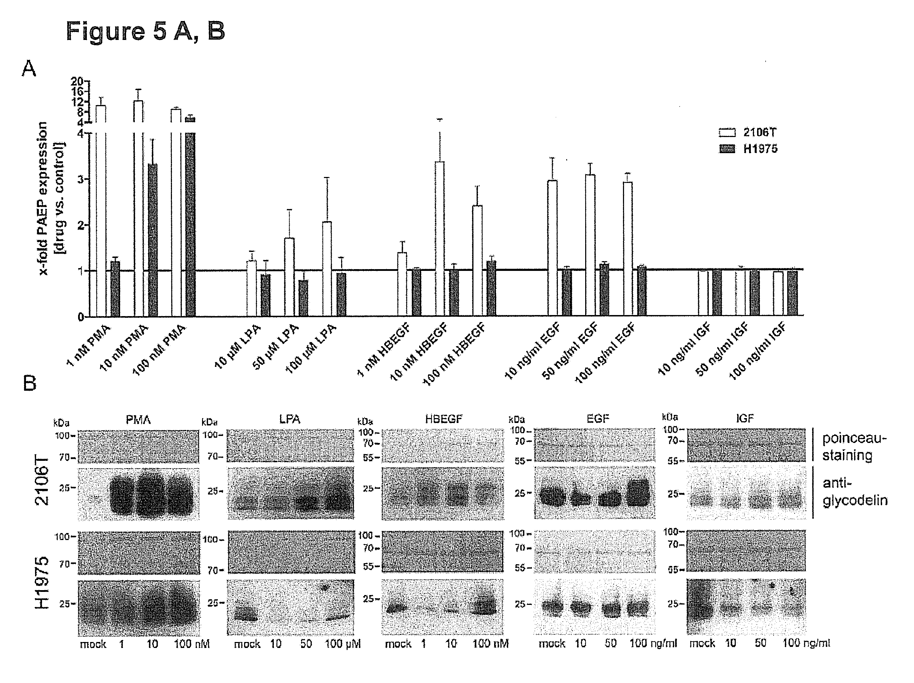

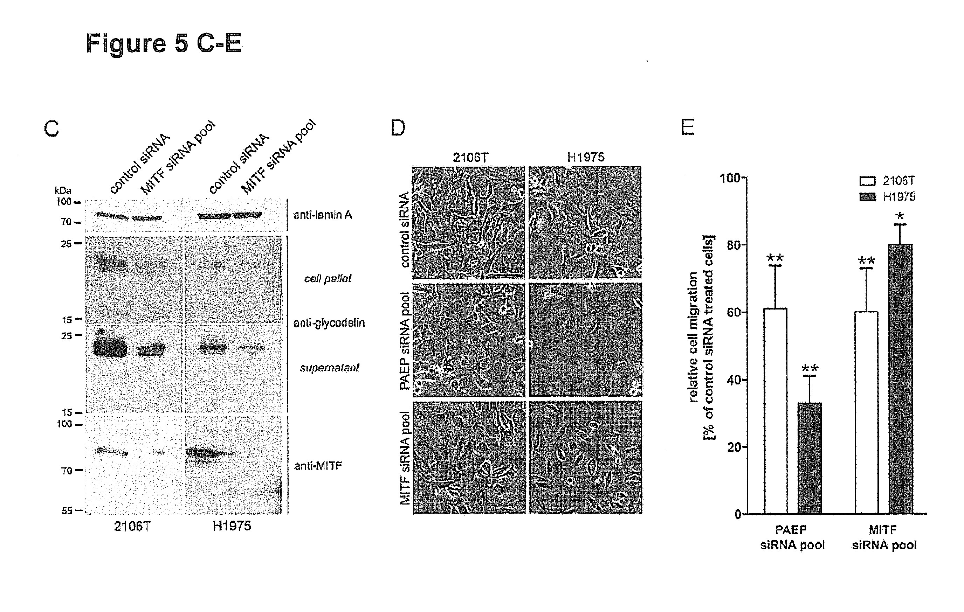

**Figure 5****.** *Glycodelin expression is differentially regulated and regulates cell migration.*
   **A and B,** cells were serum-starved overnight and treated with the indicated drugs for 24 h. Cell pellets were used for RNA isolation and *PAEP*-expression was determined using qPCR. Supernatants were collected for detection of Glycodelin by western blot. Poinceau staining was used as loading control. Western blots were repeated at least two times. Gene expression assays were repeated three times on different days.
   **C,** transcription factor *MITF* was knocked out by a pool of four *MITF*-specific siRNAs. After 72 h, medium was changed. 24 h later, supernatants as well as cells were collected and glycodelin, MITF and lamin A was detected by western blot.
   **D,** cells were treated with control siRNA, *PAEP-* or *MITF*-siRNA pool for 72 h.
   **E,** cells were treated with *PAEP-* or *MITF*-siRNA pool for 72 h and serum-starved overnight. The next day, a migration assay was performed three times on different days with at least two biological replicates each for 24 h.

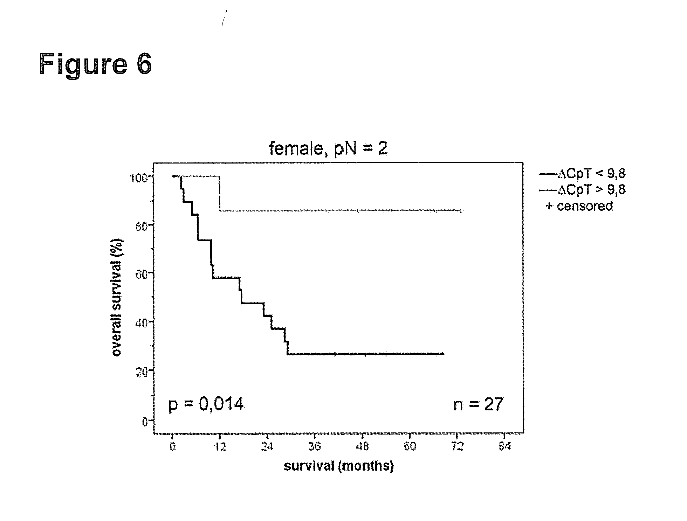

**Figure 6****.** *High expression of PAEP in tumors is strongly associated with worse survival of women with lymph node status N2.*
   Percentage overall survival is depicted for tumors with normalized Ct values <9.8 or >9.8.

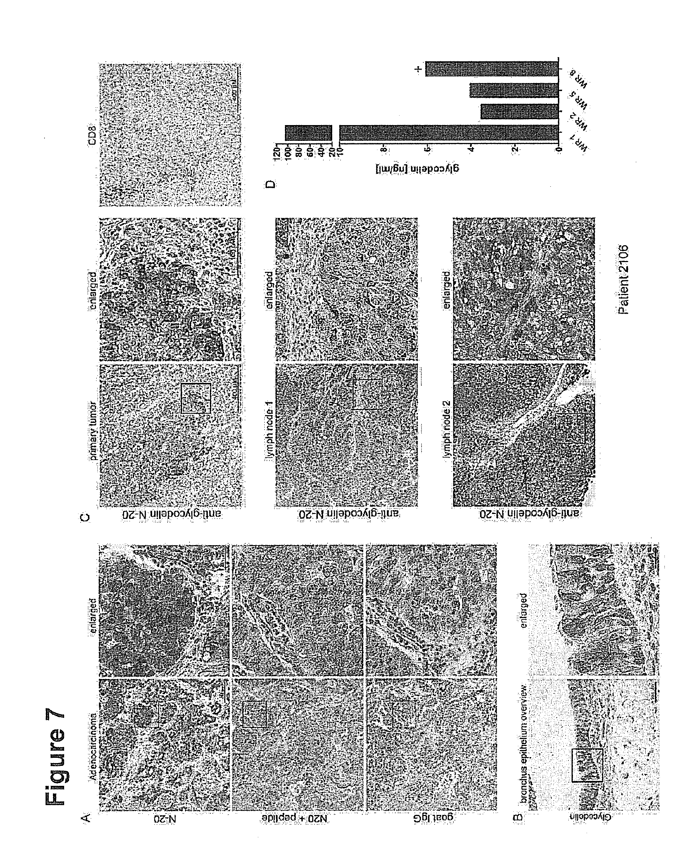

**Figure 7****.** *Glycodelin antibody N-20 binds its target specifically.*
   **A,** in parallel to normal staining procedure, glycodelin antibody N-20 antibody was either incubated with its blocking peptide for 1 h at room temperature or replaced by a normal goat IgG prior to overnight incubation.
   **B,** staining of the bronchus epithelium with polyclonal N-20 antibody.
   **C,** staining of primary tumor and affected lymph nodes of patient 2106.
   **D,** glycodelin concentration in serum of patient 2106 was quantified before surgery and during follow up ward rounds. + = tumor independent dead.

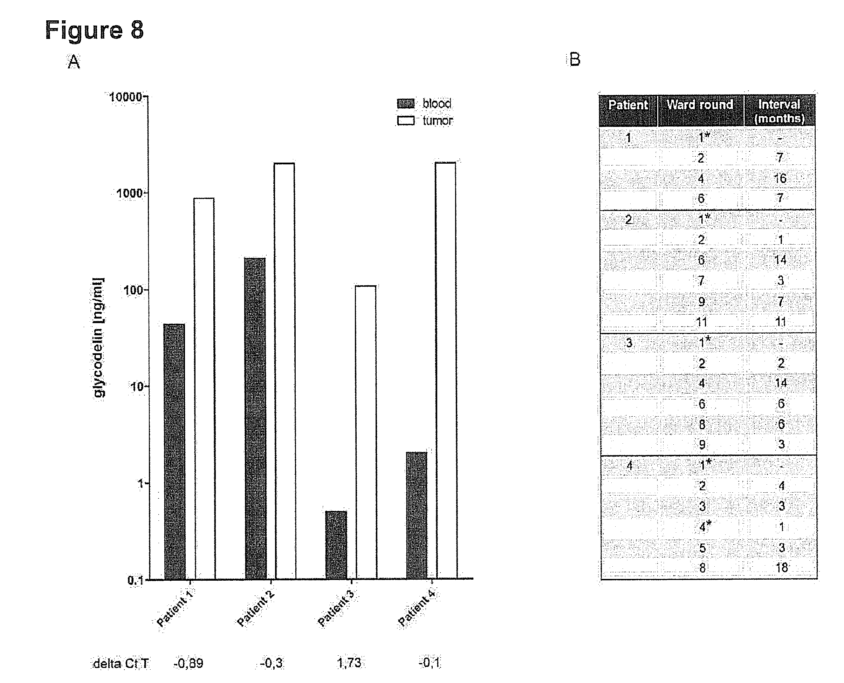

**Figure 8****.** *Intratumoral glycodelin concentration and ward round intervals.*
   **A**, serum glycodelin as well as intratumoral glycodelin levels of 4 patients with high mRNA *PAEP* expression levels were investigated (normalized Ct value <3). Tumor tissue was homogenized and Glycodelin concentration was determined with ELISA.
   **B,** intervals between blood collections.
      *= blood sample was taken before anesthesia and surgery of primary tumor and metastasis.

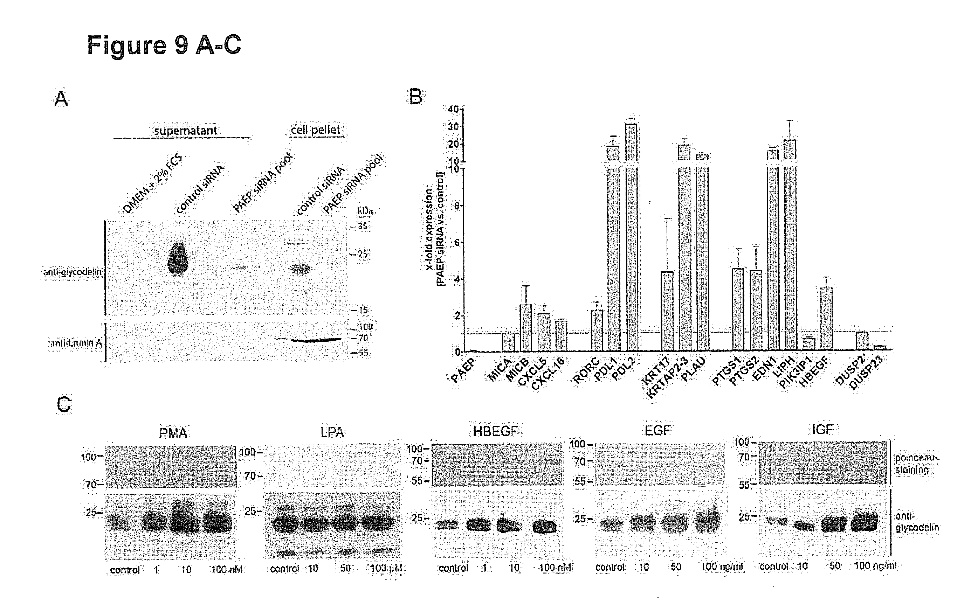

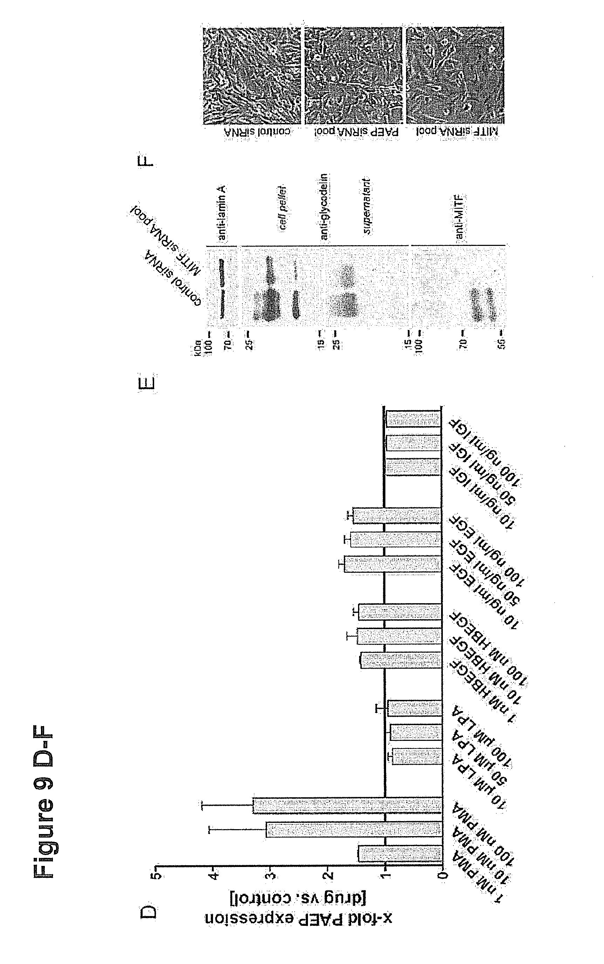

**Figure 9****.** *Glycodelin-expression and function in MeWo.*
   **A,** MeWo cells were transfected with a pool of four different *PAEP*-specific siRNAs for 72h. Medium was changed and 24 h later, supernatants as well as cells were collected and glycodelin was detected by western blot. Lamin A was used as loading control.
   **B,** after 72 h knock-out of *PAEP,* expression of indicated genes was determined by qPCR.
   **C and D,** cells were serum-starved overnight and treated with the indicated drugs for 24 h. Supernatants were collected for detection of Glycodelin by western blot. Poinceau staining was used as loading control. Cell pellets were used for RNA isolation and *PAEP*-expression was determined using qPCR. Western blots were repeated at least two times. Gene expression assays were repeated three times on different days.
   **E,** transcription factor *MITF* was knocked out by a pool of four *MITF*-specific siRNAs. After 72 h, medium was changed. 24h later, supernatants as well as cells were collected and glycodelin, MITF and lamin A was detected by western blot.
   **F,** cells were treated with control siRNA, *PAEP-* or *MITF*-siRNA pool for 72 h.

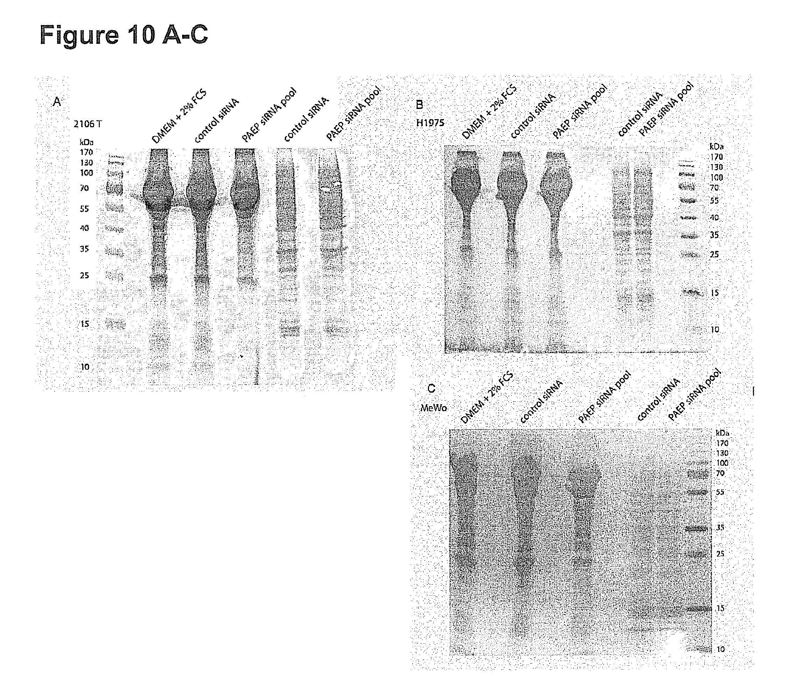

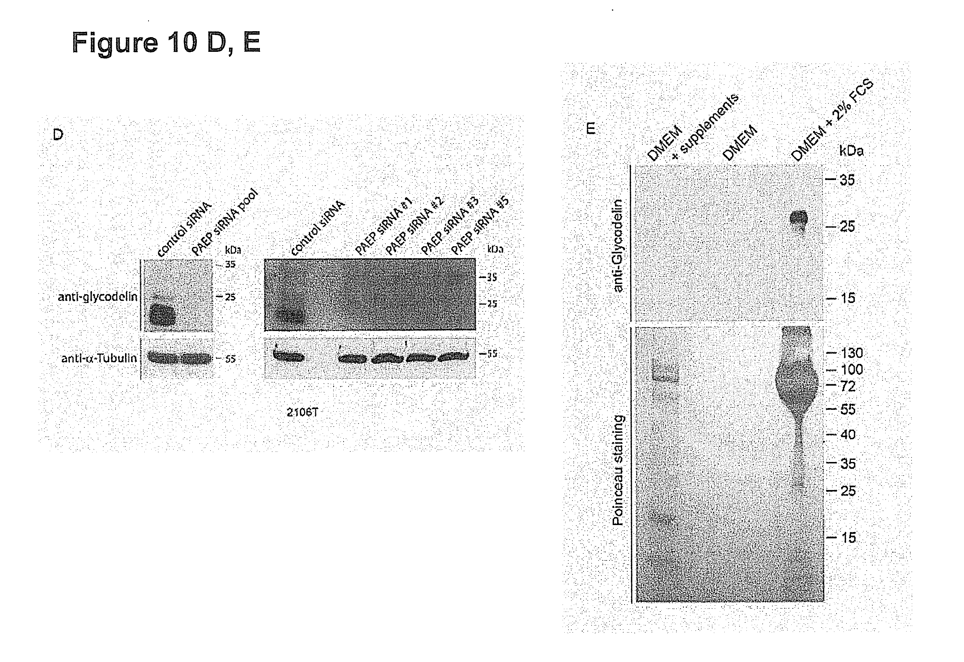

**Figure 10****.** *Controls to siRNA knockdown experiments.*
   **A-C,** Poinceau staining confirms homogenous precipitation of supernatants.
   **D,** 2106T cells were transfected for 72 h with siRNA pool or 4 different single siRNAs to knockdown *PAEP.* Tubulin was used as loading control.
   **E,** DMEM, DMEM with supplements and DMEM + 2% FCS were precipitated and used for western blot analyses of glycodelin.

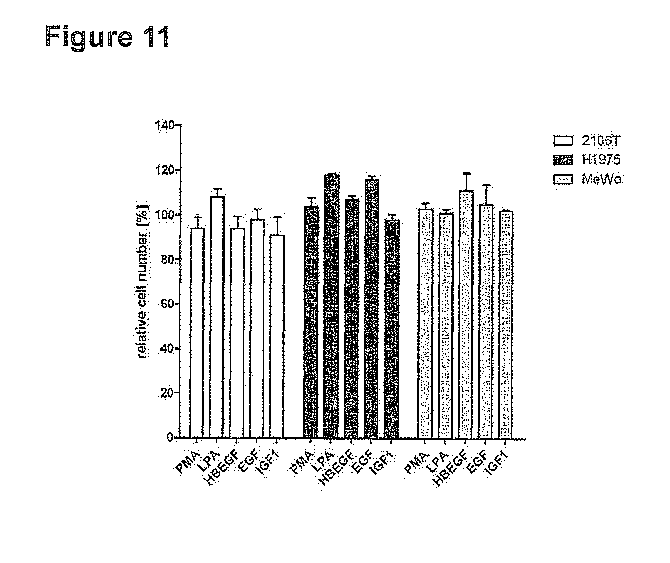

**Figure 11****.** *Toxicity controls of drug treatment.*
   Cells were serum-starved overnight and treated with indicated drugs (highest concentrations used in PAEP induction assay) for 24 hours. LDH activity was measured as a mass for relative cell number. Cell number of control treated cells was set to 100%. At least three biological repeats were used.

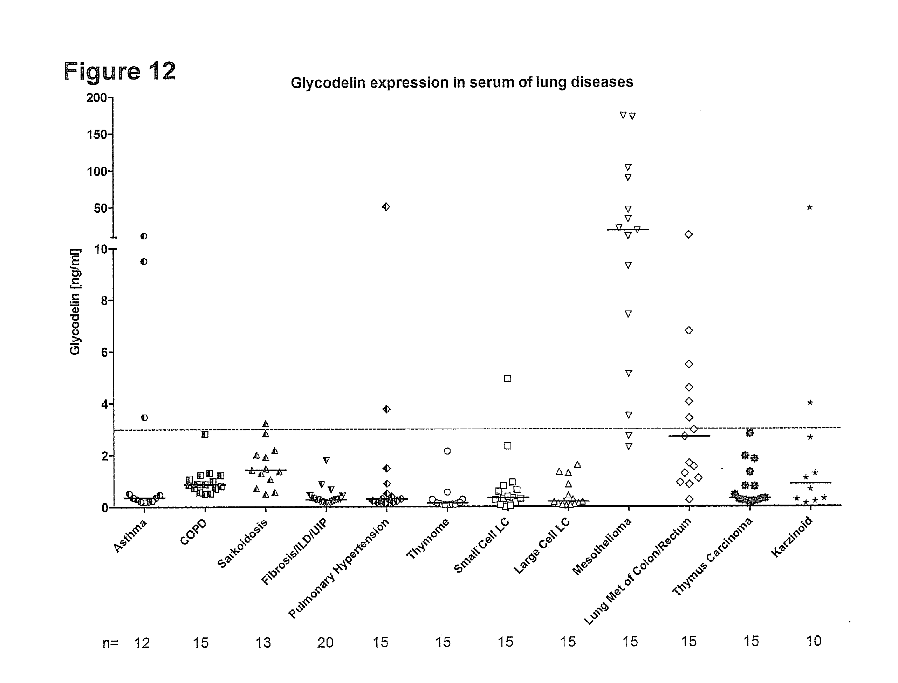

**Figure 12****.** *Glycodelin expression in serum of lung diseases.*
   Glycodelin serum concentration was determined from patients with indicated benign and malignant lung diseases.

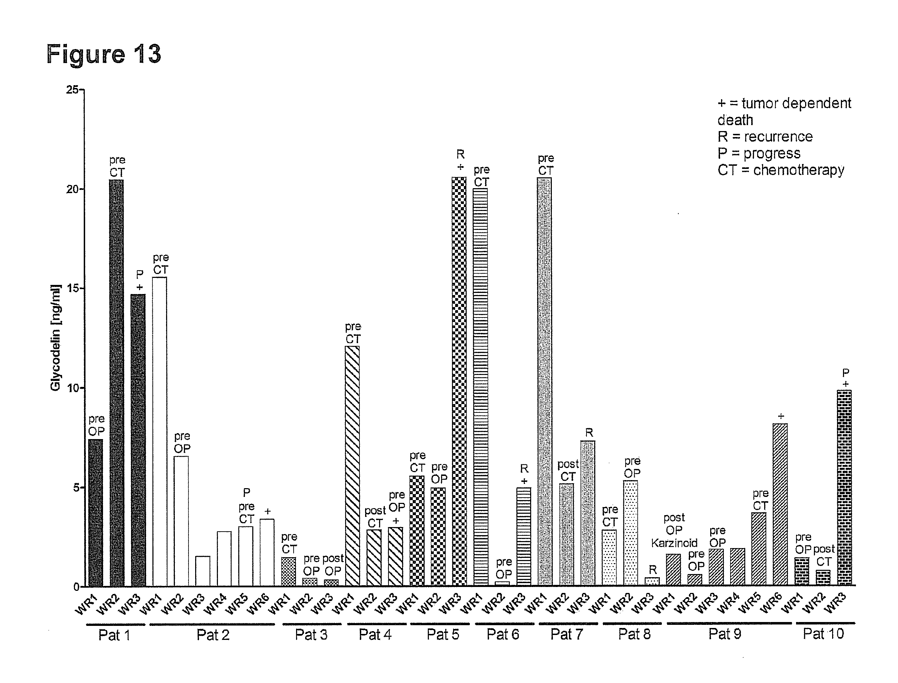

**Figure 13****.** *Monitoring of mesothelioma treatment.*
   Glycodelin concentration in serum was quantified in patients with mesothelioma in first ward round before treatment (surgery and/or chemotherapy and/or radiotherapy) and in follow up.
**Figure 14****.** *ROC-Analyses for mesothelioma patients.*
   Receiving operator analyses were performed for glycodelin serum determination of mesothelioma patients (n = 95) compared to benign patients (n = 105).

### EXAMPLES

### 1. Materials and Methods

### 1.1 Tissue sample collection, characterization and preparation

Tissue samples were provided by Lung Biobank Heidelberg, which is a member of the accredited Tissue bank of the National Center for Tumor Diseases (NCT) Heidelberg, the BioMaterialBank Heidelberg and the Biobank platform of the German Center for Lung Research (DZL). All patients gave written informed consent for the use of the tissue for research purpose. The study was approved by the local ethics committee of the University of Heidelberg (No. 270/2001). Tumor and matched distant (> 5 cm) normal lung tissue samples from NSCLC patients (n=362) who underwent resection for primary lung cancer at the Thoraxklinik at University Hospital Heidelberg, Heidelberg, Germany were collected. Tumor histology was classified according to the 3rd edition of the World Health Organization classification system (Beasley et al. 2005). Tissues were snap-frozen within 30 minutes after resection and stored at -80°C until the time of analysis. For nucleic acid isolation 10 - 15 tumor cryo sections (10 - 15 µm each) were prepared for each patient. The first and the last section of a series were stained with Hematoxylin and Eosin (H&E) and were reviewed by an experienced lung pathologist to determine the proportion of viable tumor cell, stromal cell, normal lung cell content, infiltrating lymphocytes and necrotic areas. Only samples with a viable tumor content of ≥50% were used for subsequent analyses. Matched tumor free lung tissues were removed distant to the tumor and macroscopically reviewed to be devoid of tumor.

### 1.2 Total RNA isolation and cDNA synthesis

Frozen tumor cryo sections were homogenized with the TissueLyser mixer-mill disruptor (2 x 2 min, 25 Hz, Qiagen, Hilden, Germany). Matched normal lung tissue pieces were homogenized using a Miccra D-8 rotor-stator homogenizer with DS-5/K1 (2 x 30 sec, 26.000 rpm, Art-modeme Labortechnik, Muelheim, Germany). Total RNA was isolated from tissue using an RNeasy Mini Kit (Qiagen) according to the manufacturer's instruction. For RNA isolation from cell lines, AllPrep DNA/RNA Mini Kit (Qiagen) was used. The quantity of RNA was measured with a NanoDrop ND-1000 Spectrophotometer (NanoDrop Technologies, Wilmington, MA, USA). The quality of total RNA was assessed with an Agilent 2100 Bioanalyzer and Agilent RNA 6000 Nano Kit (Agilent Technologies, Boeblingen, Germany). Total RNA was considered sufficient for further analyses if it had an RNA integrity number (RIN) of at least 8.0. Total RNA was transcribed to sscDNA with a Transcriptor First Strand cDNA Synthesis Kit (Roche) in three independent reactions. Complementary DNA synthesis was performed with anchored oligo(dT)18 primer and random hexamer primer. To ensure denaturation of possible secondary RNA structures 2 µg total RNA, 1 µl of oligo(dT)18 and 2 µl of random hexamer were incubated in a total volume of 13 µl for 10 min at 65°C, followed by a cooling step for 5 min at 4°C. Final concentration of oligo(dT)18 and random hexamers was 2,5 µM and 60 µM, respectively. Reaction was completed with 4 µl 5x reaction buffer, 0,5 µl RNase inhibitor (20 U), 2 µl of dNTPs (1 mM each) and 0,5 µl of reverse transcriptase (10 U) and filled up with water to final volume of 20 µl. Reverse transcription was performed by incubation reaction mixture for 10 min at 25°C, 60 min at 50°C and 5 min at 85°C. In addition, a reaction without RNA (no RT control) was performed as a control for possible contaminations by genomic DNA. The three independent sscDNA reaction mixtures were pooled, mixed by pipetting and separately (20 µl aliquots) stored at -80°C for further analyses.

### 1.3 Gene expression analyses

For gene expression analysis, we used the whole human genome expression microarray Affymetrix U133 Plus 2.0 (Affymetrix, Santa Clara, CA, US) covering about 47,000 transcripts and variants. The microarray experiment comprised 3 control siRNA transfected and 3 PAEP siRNA pool transfected samples per cell line. Total RNA was extracted using the RNeasy Mini Kit (Qiagen, Hilden, Germany) according to the manufacturers' instructions. Amplification of total RNA samples, subsequent labeling of the amplified RNA, hybridization, washing and scanning of the microarrays were done using the recommended operating procedure for Affymetrix GeneChip®. Preprocessing and normalization was performed as described before (Kuner et al., 2009). The microarray data were deposited into the NCBI GEO database (GSE10245). In order to select genes showing variation between the analyzed samples, the Median Average Deviation (MAD) of each gene was calculated. The genes resembling the highest 5% of all MAD values were used for further analysis, After separating the data samples into the respective groups to compare, Wilcoxon two-sided rank test was applied to select those genes below a p-value threshold of 0.05. Adjusted p-values were calculated to correct for multiple hypothesis testing using the method of Benjamini and Hochberg. However, due to the small sample size, the adjusted p-values did not reach the threshold of 0.05.

### 1.4 Quantitative Realtime PCR (qPCR)

Real-time quantitative PCR (qPCR) was performed in accordance to MIQE-guidelines (Bustin et al., 2009) using a LightCycler® 480 Real-Time PCR Instrument in 384-well plate format (Roche). Gene-specific primer and probes (Universal ProbeLibrary, Roche) were used in combination with ABsoluteTM QPCR Mix (ThermoScientific). One qPCR reaction (12 µl total reaction volume) comprised 5 µl template, 6 µl 2x QPCR master mix, 0.12 µl each forward and reverse primer, 0.12 µl probe and 0.64 µl PCR grade H2O. Final concentration of each primer was 0.2 µM, and 0.1 µM for the probe. The input amount of cDNA in one single PCR reaction corresponded to 5 ng total RNA. Technical triplicates as well as a non-template control were used to ensure the measurements. qPCR conditions were: activation of the Taq polymerase at 95°C for 15 min, followed by 45 cycles of 10 sec at 95°C (denaturation), 30 sec at 60°C (annealing) and 1 sec at 72°C (elongation). CT values were calculated with LightCycler® 480 software version 1.5 using the 2nd derivative maximum method (Roche). To evaluate differences in gene expression, a relative quantification method based on the ΔΔ CT -method was performed (Livak et al., 2001).Target genes were normalized with the mean of two housekeeping genes (ESD, Esterase D and RPS18, 40S Ribosomal Protein S18). For PCR efficiency calculation, five dilutions (corresponding to total RNA amounts from 50 ng to 5 pg (1:10 dilutions) of cDNA from QPCR Human Reference Total RNA (Agilent Technologies) were used to amplify target genes. Amplification of ESD from cDNA of QPCR Human Reference Total RNA (corresponding to 5 ng of total RNA) was performed within every qPCR run to compare CT values from different plates (internal plate calibrator). GenEx 5 software (multid Analyses) was used for determination of genes used for normalization and pre-processing of raw data (internal plate calibration, determination of PCR efficiency, calculating the mean CT value, normalization to reference genes (ACT)). To obtain the normalized, relative value (ΔΔCT), CT values of tumor tissue were compared to tumor adjacent normal tissue. For cell culture experiments, CT values of treated cells were compared to control cells.

Samples from tumor and corresponding normal tissue with a mean CT value > 38 cycles or a standard deviation > 0.3 were not included in the advanced statistical analysis. To include samples from either tumor or corresponding normal tissue with a CT value > 38 in statistical analyses, CT was set to 38. Genes were only amplified when PCR efficiency was within range from 0.9 to 1.1.

To ensure the correct amplification of the targets, amplicons from qPCR efficiency calculations (5 ng dilutions) were cloned into pJet 1.2 cloning vector with CloneJET PCR Cloning Kit (ThermoScientific) and confirmed by sequencing (Eurofins MWG GmbH). Results of qPCR were visualized with GraphPad Prism 5 (GraphPad Software). Box plots show upper and lower quartile, median, and the range from minimum to maximum.

### 1.5 Immunohistochemistry

To compare the expression of whole glycodelin and glycodelin A, the polyclonal N-20 antibody (sc-12289, Santa Cruz Biotechnology) and the monoclonal A87-B/D2 (Glycotope) antibody, raised against glycodelin A that was purified from mid-trimester amniotic fluid, were used. Paraffin-embedded tissue sections were deparaffinized with the following steps: 2x 10 min Xylol, 2x 5 min Ethanol 100%, 1x 3 min Ethanol 98%, 1x 3 min Ethanol 70%. Antigen retrieval was performed in a steamer with sodium-citrat-buffer (10mM Sodium Citrate, 0.05% Tween 20, pH 6.0) for 15 min. The following staining procedure for polyclonal anti-glycodelin antibody was performed with DAKO EnVision+ System-HRP (AEC) for mouse primary antibodies (Dako). The tissue slides were incubated overnight at 4°C with anti-Glycodelin antibody in a concentration of 2,5 µg/ml diluted in DAKO antibody diluent. A linker (mouse anti-goat IgG, #31107, ThermoScientific) antibody was used for 30 min at room temperature before tissue sections were incubated with secondary antibody for another 30 min at room temperature. Visualization of Glycodelin was performed with AEC+ Substrate-Chromogen (Dako) incubation for 8 min. To ensure specific staining, parallel staining with a normal goat IgG (sc-2028, Santa Cruz Biotechnology) and a preincubation of glycodelin antibody with the specific antigen peptide (sc-12289 P, Santa Cruz Biotechnology) for one hour at room temperature was used for general staining procedure.

Staining of monoclonal anti-Glycodelin antibody, raised against glycodelin A that was purified from mid-trimester amniotic fluid, was performed with standard New England Biolabs (NEB) IHC protocol using Signal Stain® DAB substrate kit. First antibody was diluted 1:200 in diluent reagent (NEB) and incubated overnight at 4°C. Slides were incubated the next day for 30 minutes with secondary antibody at room temperature. Staining was visualized with DAB incubation for 1-5 minutes.

Detection of CD8 was performed by National Cancer for Tumor Diseases Heidelberg (NCT) with monoclonal mouse anti-CD8 antibody (Clone C8/144B, Dako). Staining was considered with Olympus IX-71 inverted microscope. Pictures were taken with Olympus Color View II digital camera and Olympus Cell-F software (Olympus). Tiffs were assembled into figures using Photoshop CS6 (Adobe).

### 1.6 Detection of glycodelin in human sera

Human sera were collected prior to first surgery and stored at -80°C. Glycodelin level of sera was detected using an Enzyme-Linked Immunosorbent Assay kit (ELISA BS-20-30, Bioserv Diagnostics GmbH, Rostock, Germany) with 50 µl of each serum in two technical replicates. In case of progress measurements, Glycodelin was measured from sera collected during patient's routine checkup and/or before repeated surgical intervention. Glycodelin from sera of pregnant women (first trimester, kindly provided by Dr. Michael Elsaesser, University Medical Center of Heidelberg) was used as an internal positive control. The readout and standard curve were performed with ELISA Reader (Tecan Group Ltd.). Results of ELISA were visualized with GraphPad Prism 5.

### 1.7 Statistical analyses

Data of qPCR analyses were statistically analysed with SPSS 21.0 for Windows (IBM). The evaluation of discriminatory values for *PAEP* expression in tumor, that differentiated best between groups of patients with good and poor survival prognosis, was performed with the critlevel procedure (Abel et al., 19984) using ADAM statistical software package (German Cancer Research Center, DKFZ, Heidelberg, Germany). Binary variables were built using these cut-offs. The endpoint of the study was overall survival. Therefore, survival time was calculated from date of surgery until last date of contact or death. Univariate analysis of survival data was performed according to Kaplan and Meier. With the log-rank-test, significance between the groups was tested. A p-value of less than 0.05 was considered significant. Multivariate survival analysis was performed using Cox proportional hazards model. Data of ELISA were statistically analysed with GraphPad Prism 5. The non-parametric Mann-Whitney U test as well as Kruskal-Wallis test were used to investigate significant differences between the patient groups.

### 1.8 Cell culture:

The cell lines 2106T, 2106LN and 2427T were generated from human squamous cell carcinomas as described before (Gottschling et al., 2012). Analysis of "The Cancer Cell Line Encyclopedia" (Barretina et al., 2012) identified high PAEP expression in the two cell lines H1975 and MeWo. H1975 cell line was purchased from ATCC. 2106T, 2106LN, 2427T and H1975 were cultivated in DMEM/Ham's F-12 (Life technologies) with 10% fetal calf serum (FCS) (PAA) for not more than 20 passages. MEWO, MDA-MB-436, Jurkat E6.1 and THP1 were purchased from cell lines service (CLS). KHYG-1 and H460 were obtained from German Collection of Microorganisms and Cell Cultures (DSMZ). H1650, H838 and A549 were kindly provided by Prof. Dr. Ursula Klingmueller, German Cancer Research Center (DKFZ) and DNA typed by (DMSZ). Jurkat E6.1, THP1, KHYG-1, H460 and H838 were cultivated with RPMI 1640 (Life technologies) and 10% FCS. For KHYG-1 cell line 10 ng/ml IL-2 (Santa Cruz Biotechnology, sc-8438) was added to the medium. MDA-MB-436, H1650 and A549 were cultivated with DMEM/Ham's F-12 with 10% FCS, MeWo with EMEM supplemented with 2 mM L-glutamine, 1% nonessential amino acids, 1 mM sodium pyruvate and 10% FCS (premix from CLS). For indicated experiments, cell lines were cultured for a maximum of 24 hours with complete serum-free growth medium (DMEM/Ham's F-12 containing sodium selenite 30 nM (Sigma #S9133), ethanolamine 10 µM (Sigma #E0135), phosphorylethanolamine 10 µM (Sigma #P0503), Sodium Pyruvate 0.5 µM (PAA, S11-003), Adenine 0,18 mM (Sigma #A2786), HEPES 15 mM (PAA, S11-001) as well as SupplementPack for Airway Epithelia Cell Growth Medium (PromoCell, #C-39160 without Epinephrine 250 and RA-50).

### 1.9 SiRNA experiments

The following siRNAs were used (Qiagen): Hs_PAEP_1, Hs_PAEP_2, Hs_PAEP_3, Hs_PAEP_5 (Gene Solution siRNA, #1027416), Hs_MITF_1, Hs_MITF_2, Hs_MITF5, Hs_MITF_8 (Gene Solution siRNA #1027416). For the performed assays, all four siRNAs were pooled and used with a resulting RNAi concentration of 10 nm. Allstars negative control siRNA (Qiagen, #1027280) was used as non-silencing control siRNA. Cell lines 2106 T, H1975 as well as MeWo were transfected with LipofectamineTM RNAiMax according to manufacturer's instructions. 48 h after transfection, medium was changed and cells were cultured for further 24 h with DMEM supplemented with 2% FCS or serum-free growth medium with supplements. Knockdown of PAEP and MITF was controlled by qPCR and Western Blot with the corresponding antibodies. Proteins from supernatants of the cells were precipitated with concentrated trichloroacetic acid (TCA) for 30 min on ice, centrifuged for 30 min at 4°C, washed with 5% TCA and Acetone. The pellet was boiled with SDS sample buffer (0.13 M Tris-HCL pH 6.8, 10% glycerol, 4% SDS, 1.4 mM 2-Mercaptoethanol, 1% Pyronine) and used for Western Blot analyses. Anti-beta-actin, anti-lamin A or anti-alpha-tubulin were taken as loading controls (Sigma Aldrich, #A5441 and #L1293 and Santa Cruz Biotechnology, sc-5286) for intracellular protein level. Poinceau-staining was used as loading control of supernatants.

### 1.10 Migration assay

For migration assay 2106T (2.5x10⁴) and H1975 (3x10⁴) cells were seeded in a 24-well plate. The next day, cells were transfected with control siRNA, *PAEP* or *MITF* siRNA pool for 72 hours. Afterwards, cells were serum-starved overnight (16 hours), treated with 10 µg/ml Mitomycin C (Applichem) for 2 hours and detached with Accutase-solution (Promocell). 5x10⁴ cells were applied with 300 µl serum-free DMEM onto ThinCerts™ Cell Culture Inserts (Greiner bio-one) with a pore size of 8 µm. At the bottom side, 500 µl of DMEM completed with 10% fetal bovine serum was prepared. After 24 hours, cells that migrated through the membrane were detached from the underside of ThinCerts™ with Accutase-solution. Cells were washed with PBS and LDH activity as a measure for relative cell number was determined with Cytotoxicity Detection Kit (LDH) (Roche). Relative migration of control siRNA transfected cells was set to 100%. Experiments were performed three times with at least three biological replicates each.

### 1.11 Glycodelin induction

2106T (7x10⁴), H1975 (7x10⁴) and MeWo (1x10⁵) cells were seeded in a 24-well plate. The next day, cells were serum starved overnight (16 hours) and treated with indicated concentrations of PMA (Biomol, #AG-CN2), LPA (Santa Cruz, sc-222720), HBEGF (Biomol, #97560), EGF (Biomol, #50349) and IGF1 (Santa Cruz, sc-4589) for 24 hours. Supernatants were collected for western blot and cells were used for RNA isolation and qPCR analyses of *PAEP.* LDH activity was measured for cells treated with the highest drug concentrations compared to control treated cells.

### 2. Results

### 2.1 PAEP is strongly overexpressed in adeno- and squamous cell carcinoma

*PAEP* expression was investigated in 362 patient samples. Patients' characteristics are given in *Table 1. PAEP* was expressed (Ct value < 38) in 90% of all tumors, but only in 54% of normal tissue *(Table 2).* 144 patients (40%) exclusively expressed *PAEP* in tumor but not in normal tissue. 26 (7%) patients did express *PAEP* neither in tumor nor in normal tissue and were excluded from further analyses.

**Table 1 Patients' characteristics (n- 362)**

| Parameter | n | Percentage (%) |
|---|---|---|
| *Median Age* | 65 (38-88) | |
| | | |
| Gender | | |
| Male | 250 | 69 |
| Female | 112 | 31 |
| | | |
| *Histology* | | |
| Adeno | 211 | 58 |
| Squamous | 151 | 42 |
| | | |
| *Pathological stage* | | |
| 1a | 37 | 10 |
| 1b | 90 | 25 |
| 2a | 70 | 19 |
| 2b | 51 | 14 |
| 3a | 105 | 29 |
| 3b | 9 | 2 |
| | | |
| *ECOG* | | |
| 0 | 320 | 88 |
| 1 | 32 | 9 |
| 2 | 4 | 1 |
| n.d. | 8 | 2 |
| | | |
| *Treatment* | | |
| OP | 212 | 59 |
| OP + CT | 100 | 28 |
| OP + RT | 13 | 4 |
| OP + CT + RT | 37 | 10 |
| | | |
| *Survival* | | |
| Tumor dependent death | 92 | 25 |
| Tumor independent death | 21 | 6 |
| Unknown death | 12 | 3 |
| Alive | 237 | 65 |

**Table 2 PAEP expression in lung tissue**

| *qPCR statistics* | | |
|---|---|---|
| Parameter | n | Percentage (%) |
| *PAEP-Expression* | 362 | 100 |
| | | |
| tumor | 325 | 90 |
| normal | 196 | 54 |
| exclusively in tumor | 144 | 40 |
| exclusively in normal | 15 | 4 |
| expressed in both | 181 | 50 |
| not expressed | 26 | 7 |
| *Multivariate analysis of Overall Survival* | | |

| Variable | Hazard Ratio (95% CI) | *p* |
|---|---|---|
| *PAEP* tumor (ΔCt<9.8 vs. ΔCt>9.8) | 1.31 (0.88-1.95) | 0.19 |
| Histology (adeno vs. squamous) | 1.11 (0.73-1.69) | 0.62 |
| **Gender (female vs. male)** | **0.59 (0.38-0.93)** | **0.02** |
| pN (1 vs. 0) | 1.40 (0.89-2.20) | 0.15 |
| **pN (2 vs. 0)** | **2.55 (1.69-3.86)** | **<0,001** |

Comparison of tumor and normal tissue showed that *PAEP* expression level was elevated in more than 80% of all squamous cell carcinomas and more than 75% of all adenocarcinoma compared to normal tissue (Figure 1A). No significant gender specific expression of *PAEP* was observed between male and female patients in our study. The median overexpression of *PAEP* in adenocarcinoma was significant higher than in squamous cell carcinoma (p < 0.0001, Figure 1B). Both cancer types showed a high expression range of *PAEP* in tumor tissue from 20-fold down regulation up to a more than 8000-fold expression compared to paired normal tissue. Regarding the different stages of patients' disease, median *PAEP* expression was up regulated in stage 2 and 3 for both, adenocarcinoma and squamous cell carcinoma (Figure 1C). While *PAEP* was expressed in a broad range of more than 16 cycles in tumor tissue, normal tissue showed a lower expression with less variation (Figure 1D), suggesting a tumor specific function of glycodelin.

### 2.2 Expression of PAEP in NSCLC is a prognostic factor in females

Statistical analyses were performed with patients expressing *PAEP* in NSCLC (n = 336). Patients with SQCC and increasing mRNA levels of *PAEP* presented a reduced overall survival rate (Figure 1E). However, the difference in survival was not significant (p=0.139). In contrast, the intratumoral expression level of *PAEP* had no influence on the prognosis of adenocarcinoma patients (Figure 1F). Survival of NSCLC patients was strongly associated with gender. Females had a significant reduced overall survival rate when expressing a high *PAEP* level (p = 0.014, Figure 1G), while there was no correlation between expression level and survival in male patients (Figure 1H). Further analyses indicated that lymph node status is a prognostic factor in women (p = 0,016, Figure 6).

In a multivariate analysis including *PAEP* expression, histology, gender and lymph node status, only gender (female vs. male) and the lymph node status (pN 2 vs. 0) were significant prognostic factors (Table 2). *PAEP* expression (high vs. low) had a moderate increased hazard ratio but only tends to be a prognostic factor.

### 2.3 Glycodelin as well as immunosuppressive glycodelin A is expressed in NSCLC tissue

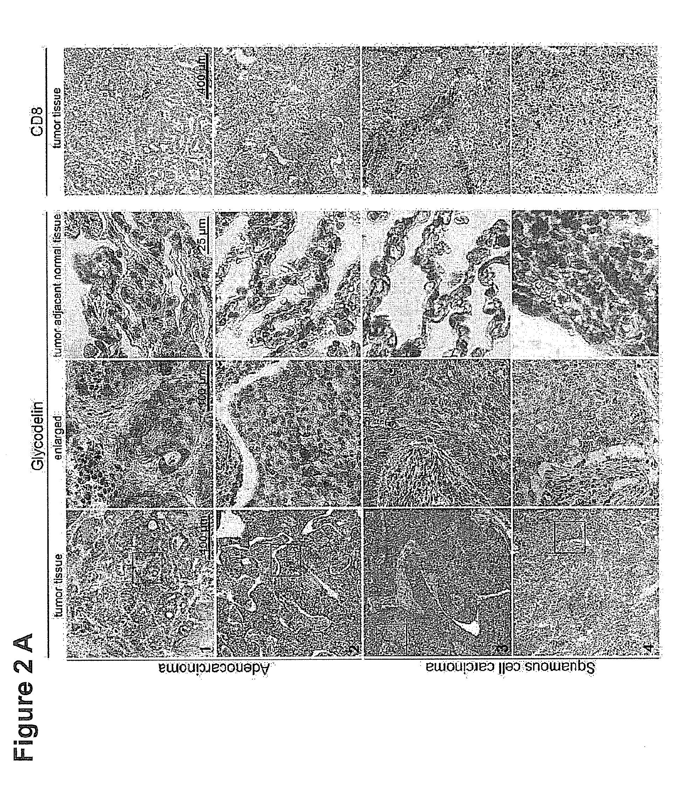

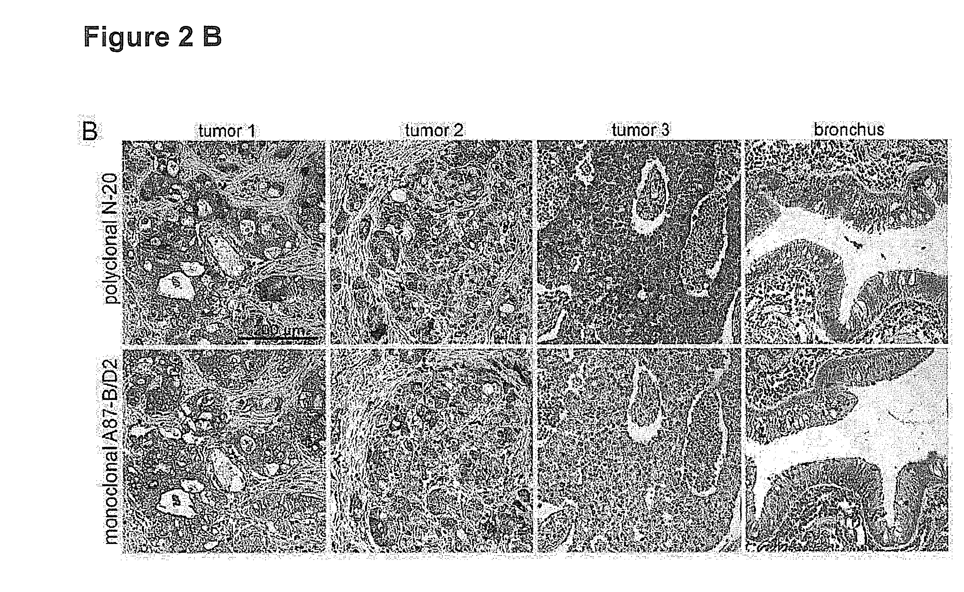

To verify the glycodelin protein expression in lung cancer, immunohistochemistry was performed on FFPE tissue slides derived from patients that were prior shown to have a high glycodelin mRNA expression. Four representative samples (two adenocarcinomas, two squamous cell carcinomas) are shown in Figure 2. The expression pattern of glycodelin varied between the NSCLC subtypes. In general, Glycodelin staining showed higher heterogeneity in adenocarcinoma (Figure 2A, patient 1 and 2). In contrast, glycodelin was expressed more homogenously but in a lower amount in almost all tumor cells of squamous cell carcinomas (Figure 2A, patient 3 and 4). Antibody specificity was tested and validated in controls using blocking peptide (Figure 7A).

To investigate the expression of immunosuppressive glycodelin A compared to total glycodelin in lung cancer, an monoclonal antibody raised against glycodelin A was used. While total glycodelin was expressed by most tumor cells (Figure 2B, upper panel), we observed a different staining pattern with the anti-glycodelin A antibody (Figure 2B, lower panel). Some signals of both antibodies were completely overlapping (first tumor). In some cases glycodelin A expression differed from total glycodelin (second tumor) or could not be detected (third tumor).

Normal lung alveolar cells showed poor expression of glycodelin in qPCR (Figure 2A, third columns). A prominent glycodelin expression could be detected in respiratory epithelial cells of bronchus using the polyclonal antibody (Figure 7B) but not with the monoclonal antibody (Figure 2B, last column). All stained tumor tissues were strongly infiltrated by CD8-positive T cells (Figure 2A, last column). Glycodelin expression could also be observed in tumor infiltrated lymph nodes, as shown for patient 2106, the donor for the in-house generated tumor cell line 2106T (Figure 7C).

### 2.4 Secreted glycodelin can be detected in serum of NSCLC patients with primary tumor, recurrence or metastatic spread

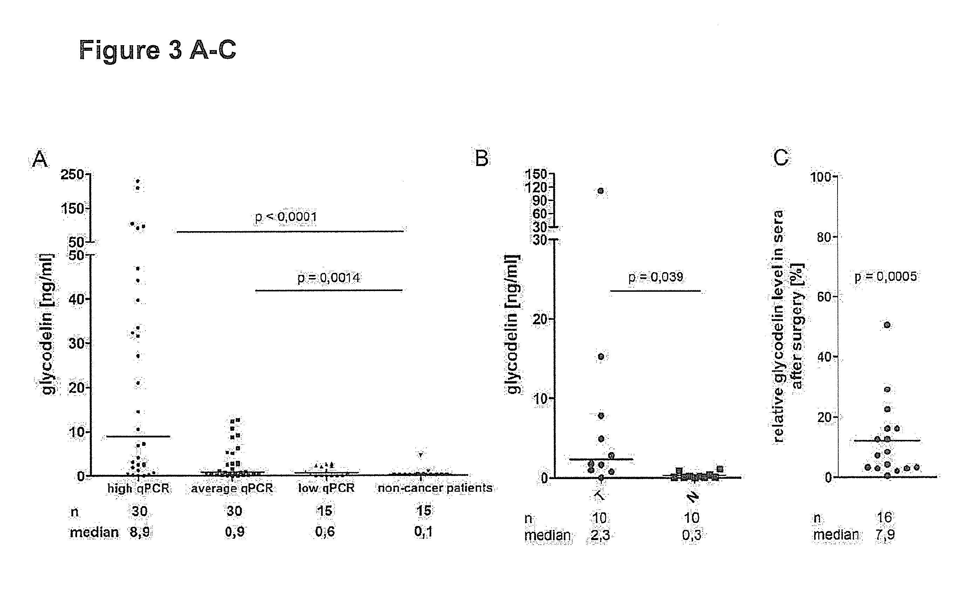

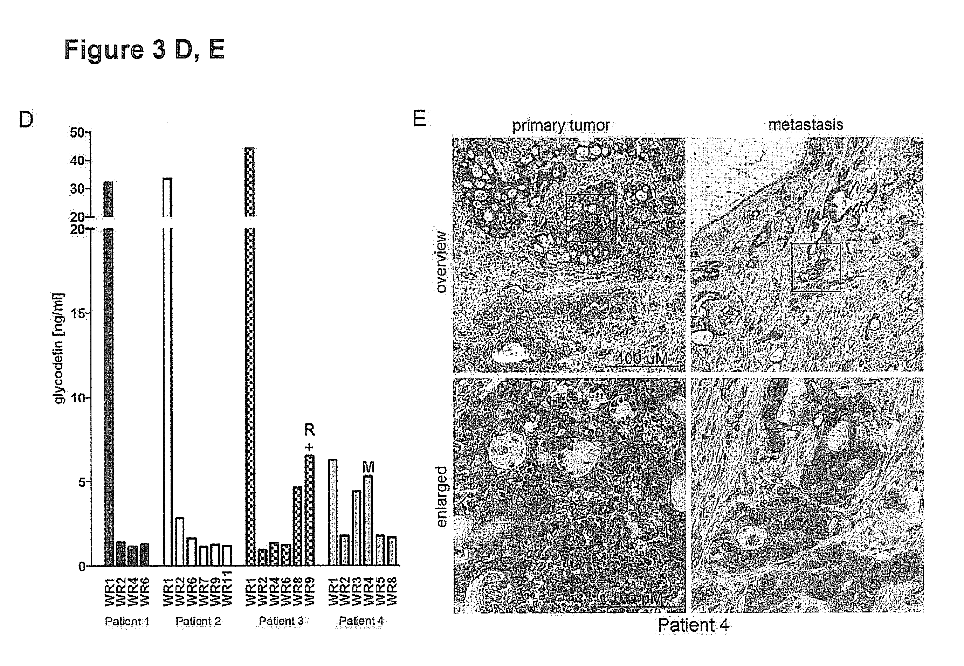

Since glycodelin is known to be secreted, we investigated the possibility to detect the protein in serum of cancer and non-cancer patients. Initially, patients were divided in subgroups of high (ΔCt<3, n = 30), average (6.8<ΔCt< 8.5, n = 30) and low *PAEP* mRNA expression (ΔCt>13, n = 15) by qPCR data Serum samples, which were collected prior to surgery, were tested for glycodelin. Patients with benign hamartomas (n = 15) were included as a non-cancer control. The highest amounts of glycodelin were found in serum of patients that had a high gene expression *of PAEP* (Figure 3A) with a median level of 8.9 ng/ml and a maximum of 228.4 ng/ml. The serum glycodelin levels from high and average *PAEP* mRNA expressing patients were significantly increased compared to glycodelin levels from non-cancer patients. Serum glycodelin levels were not detectable in some patients with high *PAEP* mRNA expression (Figure 3A, first column). This was especially true for patients with smaller tumors (data not shown). Therefore, we homogenized tumors of four patients (two with high tumor mRNA and high serum ELISA level and two with high tumor mRNA and low serum ELISA level) to investigate the intratumoral glycodelin level (Figure 8A). In all four patients, high PAEP levels could be detected despite of low serum concentrations. Glycodelin concentrations in homogenates of randomly selected tumors from 10 adenocarcinoma patients were significant higher than from paired normal tissue (Figure 3B). To further substantiate the tumor as the source of glycodelin, serum glycodelin levels were measured after complete tumor resection by surgery. As displayed in Figure 3C, the relative glycodelin levels in serum dropped down significantly after resection of the tumor (82.1 % reduction). In a further approach, serum glycodelin level was measured during clinical follow-up of patients. In accordance to Figure 3C, all four patients showed a strong decrease of glycodelin secretion in second ward round after tumor resection (Figure 3D). Glycodelin level of relapse-free patients 1 and 2 remained stable around 1.5 ng/ml following surgery. In contrast we found increasing glycodelin levels in case of recurrence or metastatic spread (patient 3 and 4). Immunohistochemistry of primary tumor confirmed glycodelin expression of patient 4 (Figure 3D). Patient 2106, the donor of used cell line, showed an increasing glycodelin serum concentration during follow-up until tumor independent death (Figure 7D). The possibility to detect glycodelin in patients' sera suggests that it might be meaningful to determine glycodelin level in follow-ups to observe possible recurrence of lung cancer or metastasis.

### 2.5 Knockdown of glycodelin in cancer cell lines leads to a reorganization of genes involved in tumor immunosurveillance and environment modulation

The role of glycodelin as a regulator of the immune system is well characterized during pregnancy (Lee et al., 2011) and hormone related cancer (Seppala et al., 2009).

To investigate a functional role of glycodelin in NSCLC, *PAEP* was silenced by siRNA-transfection in two NSCLC cell lines (Figure 4A and B) and in MeWo cell line (Figure 9A). Down regulation was observed intracellular and for secreted glycodelin. For siRNA transfection, a pool of four different *PAEP* siRNAs was used. Every single siRNA strongly reduced glycodelin expression (Figure 10D). Interestingly, culture medium with 2% fetal calf serum (FCS) alone caused an antibody signal (Figure 4A and B, first lane). This signal resulted from FCS since culture medium without supplements as well as complete serum-free growth medium did not show this band (Figure 10E). MeWo cell line showed the highest mRNA expression of *PAEP* (data not shown) and glycodelin secretion of several investigated cell lines (Figure 4C). Beside 2106 T, a lung squamous cell carcinoma cell line and H1975, an adenocarcinoma cell line, we also found a high secretion of glycodelin in the supernatant of large cell carcinoma cell line H460 and the breast cancer derived cell line MDA-MB-436. No secretion was observed for monocytic cell lines THP1 and Jurkat as well as natural killer cell line KHYG-1.

Many studies of glycodelin A stated immunosuppressive functions. To get further insight into potential molecular functions of glycodelin in tumor cells, we performed microarray gene expression profiles of the three cell lines 2106T, H1975 and MeWo after silencing of *PAEP.* The most regulated genes were validated with qPCR. Results are illustrated in Figure 4D and Figure 9. The knockdown of *PAEP* led to a strong upregulation of immune system regulating ligands (MIC proteins, CXCL5, CXCL16 and CD83, PDL1/2), to a reorganization of cytoskeleton (CLDN1, KRT17 and KRTAP2-3) and cell matrix (PLAU, MMP9) as well as to an increase of signal pathway regulating proteins (e.g. EDN1, PTGS1/2, HBEGF). These data indicate that glycodelin expression is involved in the regulation of immune defense mechanisms.

### 2.6 Glycodelin expression is differentially regulated and essential for cell migration

Early studies implicate an induction of glycodelin expression by phorbol myristate acetate (PMA) (Morrow et al., 1994) and lysophosphatidic acid (LPA) (Ramachandran et al., 2002) in cancer cell lines. In our study we found that induction of glycodelin is differentially regulated in the focused cancer cell lines (Figure 5A and B and Figure 9C and D). While PMA stimulates the expression and the secretion of glycodelin in 2106T and H1975, LPA led to an increase of glycodelin secretion in 2106T but to a decrease in H1975 while it has no influence on glycodelin secretion in MeWo. The treatment with heparin-binding EGF-like growth factor (HBEGF) as well as epidermal growth factor (EGF) enhanced glycodelin secretion in 2106T and MeWo cells, but not in H1975. In contrast, the insulin-like growth factor 1 (IGF-I) did not affect the mRNA level of *PAEP* but led to an increased release of glycodelin to the supernatant in MeWo and to a decreased release in H1975. LDH-activity was measured to determine relative cell number and exclude increased glycodelin amounts in supernatant as a result of an increased cell number (Figure 10B). Cancer cell lines, which did not express glycodelin (e.g. H838, A549, see Figure 4C), were also able to express *PAEP* after stimulation with PMA but in a very low expression level (data not shown). Treatment of 2106T, H1975 and MeWo with relaxin-1/-2, prostaglandins E1/E2/I2/F2, endothelin-1, and progesterone as well as hepatocyte growth factor had no influence on glycodelin mRNA expression and secretion (data not shown).

Previous studies demonstrated a regulation of glycodelin expression by microphtalmia-associated transcription factor (MITF) in melanoma cells (Ren et al., 2011). Silencing of MITF led to a decreased glycodelin expression and secretion in focused cell lines (Figure 5C and Figure 9E). We observed a reorganization of cell structure after knockdown of both, *PAEP* and *MITF* (Figure 5D and Figure 9F) in 2106T, H1975 and MeWo cells. Pseudopodia seemed to be shortened in *PAEP* and *MITF* siRNA transfected cells. Silencing of *PAEP* resulted in an approximately 40% reduction of migration for 2106 T and 65% reduction for H1975 (Figure 5E). MeWo cells did not show any migration in this assay.

### 3. Glycodelin levels in serum of various lung diseases

Glycodelin serum concentration were measured in patients with various lung diseases (Figure 12). High glycodelin serum concentrations were especially found in malignant diseases. Beside patients with mesothelioma, glycodelin serum concentration was increased in patients with lung metastases of a previous colon or rectum carcinoma and some patients with lung carcinoid. Glycodelin level was poorly elevated in patients with small cell lung cancer, large cell lung cancer and thymus carcinoma. In benign diseases, higher glycodelin concentrations were only detected in asthma and thymoma.

### 4. Monitoring of mesothelioma treatment

Since especially patients with malignant mesothelioma showed high glycodelin serum concentrations, serum of follow up ward rounds was tested for glycodelin expression. Figure 13 indicates that glycodelin serum concentration often corresponded with therapy state. For 7 of the 10 patients, glycodelin concentration decreased after chemotherapy treatment and fitted with the radiological observations of tumor response to therapy (data not shown) (Patients 1-7). Serum concentrations were often increased during tumor progression or recurrence (Patients 2, 4, 5, 6, 7, 9 and 10). ROC analyses of glycodelin serum concentrations between benign and mesothelioma patients resulted in an area under the curve of 88.7% (Fig. 14). 95% specificity was reached with a cut off of about 3.5 ng/ml and a sensitivity of 53.7%. The optimal cut-off of 1.1 ng/ml resulted in a specificity of 79% and a sensitivity of 84.2%.

The features disclosed in the foregoing description, in the claims and/or in the accompanying drawings may, both separately and in any combination thereof, be material for realizing the invention in diverse forms thereof.

### REFERENCES

Abel U, Berger J, Wiebelt H. CRITLEVEL: an exploratory procedure for the evaluation of quantitative prognostic factors. Methods of information in medicine. 1984;23:154-6.

Alok A, Karande AA. The role of glycodelin as an immune-modulating agent at the feto-maternal interface. Journal of reproductive immunology. 2009;83:124-7.

Al-Shibli KI, Donnem T, Al-Saad S, Persson M, Bremnes RM, Busund LT. Prognostic effect of epithelial and stromal lymphocyte infiltration in non-small cell lung cancer. Clinical cancer research : an official journal of the American Association for Cancer Research. 2008; 14:5220-7.

Barretina J, Caponigro G, Stransky N, Venkatesan K, Margolin AA, Kim S, et al. The Cancer Cell Line Encyclopedia enables predictive modelling of anticancer drug sensitivity. Nature. 2012;483:603-7.

Beasley, M. B., E. Brambilla and W. D. Travis (2005). "The 2004 World Health Organization classification of lung tumors." Semin Roentgenol 40(2): 90-97:
Bustin SA, Benes V, Garson JA, Hellemans J, Huggett J, Kubista M, et al. The MIQE guidelines: minimum information for publication of quantitative real-time PCR experiments. Clinical chemistry. 2009;55:611-22.

Darash-Yahana M, Gillespie JW, Hewitt SM, Chen YY, Maeda S, Stein I, et al. The chemokine CXCL16 and its receptor, CXCR6, as markers and promoters of inflammation-associated cancers. PloS one. 2009;4:e6695.

Davies M. New modalities of cancer treatment for NSCLC: focus on immunotherapy. Cancer management and research. 2014;6:63-75.

Giaccone G, Herbst RS, Manegold C, Scagliotti G, Rosell R, Miller V, et al. Gefitinib in combination with gemcitabine and cisplatin in advanced non-small-cell lung cancer: a phase III trial--INTACT 1. Journal of clinical oncology : official journal of the American Society of Clinical Oncology. 2004;22:777-84.

Gottschling S, Jauch A, Kuner R, Herpel E, Mueller-Decker K, Schnabel PA, et al. Establishment and comparative characterization of novel squamous cell non-small cell lung cancer cell lines and their corresponding tumor tissue. Lung cancer. 2012;75:45-57.

Govindan R, Ding L, Griffith M, Subramanian J, Dees ND, Kanchi KL, et al. Genomic landscape of non-small cell lung cancer in smokers and never-smokers. Cell. 2012;150:1121-34.

Groh V, Rhinehart R, Secrist H, Bauer S, Grabstein KH, Spies T. Broad tumor-associated expression and recognition by tumor-derived gamma delta T cells of MICA and MICB. Proceedings of the National Academy of Sciences of the United States of America. 1999;96:6879-84.

Jessmon P, Leach RE, Armant DR. Diverse functions of HBEGF during pregnancy. Molecular reproduction and development. 2009;76:1116-27

Kao LC, Tulac S, Lobo S, Imani B, Yang JP, Germeyer A, et al. Global gene profiling in human endometrium during the window of implantation. Endocrinology. 2002;143:2119-38.

Kuner R, Muley T, Meister M, Ruschhaupt M, Buness A, Xu EC, et al. Global gene expression analysis reveals specific patterns of cell junctions in non-small cell lung cancer subtypes. Lung cancer. 2009;63:32-8.

Kunert-Keil C, Jeschke U, Simms G, Kasper M. Increased expression of glycodelin mRNA and protein in rat lungs during ovalbumin-induced allergic airway inflammation. Histochemistry and cell biology. 2009;131:383-90.

Kunert-Keil C, Steinmuller F, Jeschke U, Gredes T, Gedrange T. Immunolocalization of glycodelin in human adenocarcinoma of the lung, squamous cell carcinoma of the lung and lung metastases of colonic adenocarcinoma. Acta histochemica. 2011;113:798-802.

Lam KK, Chiu PC, Chung MK, Lee CL, Lee KF, Koistinen R, et al. Glycodelin-A as a modulator of trophoblast invasion. Human reproduction. 2009;24:2093-103.

Lee CL, Lam KK, Koistinen H, Seppala M, Kurpisz M, Fernandez N, et al. Glycodelin-A as a paracrine regulator in early pregnancy. Journal of reproductive immunology. 2011;90:29-34.

Livak KJ, Schmittgen TD. Analysis of relative gene expression data using real-time quantitative PCR and the 2(-Delta Delta C(T)) Method. Methods. 2001;25:402-8.

Morris HR, Dell A, Easton RL, Panico M, Koistinen H, Koistinen R, et al. Gender-specific glycosylation of human glycodelin affects its contraceptive activity. The Journal of biological chemistry. 1996;271:32159-67.

Morrow DM, Xiong N, Getty RR, Ratajczak MZ, Morgan D, Seppala M, et al. Hematopoietic placental protein 14. An immunosuppressive factor in cells of the megakaryocytic lineage. The American journal of pathology. 1994;145:1485-95.

Motohashi S, Ishikawa A, Ishikawa E, Otsuji M, Iizasa T, Hanaoka H, et al. A phase I study of in vitro expanded natural killer T cells in patients with advanced and recurrent non-small cell lung cancer. Clinical cancer research : an official journal of the American Association for Cancer Research. 2006;12:6079-86.

Ramachandran S, Ramaswamy S, Cho C, Parthasarathy S. Lysophosphatidic acid induces glycodelin gene expression in cancer cells. Cancer letters. 2002;177:197-202.

Ren S, Liu S, Howell PM, Jr., Zhang G, Pannell L, Samant R, et al. Functional characterization of the progestagen-associated endometrial protein gene in human melanoma. Journal of cellular and molecular medicine. 2010;14:1432-42.

Ren, S., P. M. Howell, Jr., Y. Han, J. Wang, M. Liu, Y. Wang, G. Quan, W. Du, L. Fang and A. I. Riker (2011). Overexpression of the progestagen-associated endometrial protein gene is associated with microphthalmia-associated transcription factor in human melanoma. Ochsner J 11(3): 212-219.

Rosell R, Carcereny E, Gervais R, Vergnenegre A, Massuti B, Felip E, et al. Erlotinib versus standard chemotherapy as first-line treatment for European patients with advanced EGFR mutation-positive non-small-cell lung cancer (EURTAC): a multicentre, open-label, randomised phase 3 trial. The lancet oncology. 2012;13:239-46.

Rosell R, Bivona TG, Karachaliou N. Genetics and biomarkers in personalisation of lung cancer treatment. Lancet. 2013;382:720-31.

Salmon H, Franciszkiewicz K, Damotte D, Dieu-Nosjean MC, Validire P, Trautmann A, et al. Matrix architecture defines the preferential localization and migration of T cells into the stroma of human lung tumors. The Journal of clinical investigation. 2012;122:899-910.

Schneider T, Kimpfler S, Warth A, Schnabel PA, Dienemann H, Schadendorf D, et al. Foxp3(+) regulatory T cells and natural killer cells distinctly infiltrate primary tumors and draining lymph nodes in pulmonary adenocarcinoma. Journal of thoracic oncology : official publication of the International Association for the Study of Lung Cancer. 2011;6:432-8. Seppala M, Koistinen H, Koistinen R, Hautala L, Chiu PC, Yeung WS. Glycodelin in reproductive endocrinology and hormone-related cancer. European journal of endocrinology / European Federation of Endocrine Societies. 2009;160:121-33.

Sharma S, Yang SC, Zhu L, Reckamp K, Gardner B, Baratelli F, et al. Tumor cyclooxygenase-2/prostaglandin E2-dependent promotion of FOXP3 expression and CD4+ CD25+ T regulatory cell activities in lung cancer. Cancer research. 2005;65:5211-20.

Soni C, Karande AA. Glycodelin-A interferes with IL-2/IL-2R signalling to induce cell growth arrest, loss of effector functions and apoptosis in T-lymphocytes. Human reproduction. 2012;27:1005-15.

Terme M, Ullrich E, Delahaye NF, Chaput N, Zitvogel L. Natural killer cell-directed therapies: moving from unexpected results to successful strategies. Nature immunology. 2008;9:486-94.

Yu Z, Boggon TJ, Kobayashi S, Jin C, Ma PC, Dowlati A, et al. Resistance to an irreversible epidermal growth factor receptor (EGFR) inhibitor in EGFR-mutant lung cancer reveals novel treatment strategies. Cancer research. 2007;67:10417-27.

Zadran S, Remacle F, Levine RD. miRNA and mRNA cancer signatures determined by analysis of expression levels in large cohorts of patients. Proceedings of the National Academy of Sciences of the United States of America. 2013;110:19160-5.

## Claims

1. Glycodelin for use in the diagnosis, prognosis and/or treatment monitoring of a lung disease,
wherein the lung disease is selected from thoracic malignancies, mesothelioma, lung metastasis from distant malignant diseases and benign thoracic diseases.

2. The glycodelin for use according to claim 1, wherein a thoracic malignancy is lung cancer which is selected from non-small cell lung cancer (NSCLC) (such as adenocarcinoma, squamous cell carcinoma, large-cell lung carcinoma), small cell lung cancer (SCLC), lung carcinoid.

3. The glycodelin for use according to claim 1 or 2, comprising determining the presence of glycodelin in serum, plasma and/or tissue, such as tumor tissue.

4. The glycodelin for use according to claim 3, wherein determining the presence of glycodelin comprises
- determining presence / expression of glycodelin mRNA,
preferably via quantitative detection of the mRNA,
and/or
- determining presence / expression of glycodelin
preferably via immune assay(s) or immunohistochemistry, such as by using specific anti-glycodelin antibodies.

5. The glycodelin for use according to claim 3 or 4, wherein glycodelin is not detectable in normal lung parenchyma,
and glycodelin is detectable in bronchial epithelium of the lung and in tumor tissue, preferably glycodelin is increased in bronchial epithelium of the lung and in tumor tissue compared to normal lung parenchyma or glycodelin is above a threshold (value) or cut-off (value).

6. The glycodelin for use according to any of the preceding claims, wherein diagnosis of a lung disease comprises the early diagnosis of the lung disease,
and/or wherein prognosis of a lung disease comprises assessment of progression-free survival, disease-free survival and overall survival,
and/or wherein treatment monitoring comprises determining the presence of glycodelin over time,
such as before, during and/or after therapy (such as surgery, chemo-, immune- and/or radiotherapy; or combined modality treatment) and/or during follow-up after therapy.

7. The glycodelin for use according to claim 6, wherein a therapy is found successful, if the glycodelin level during and/or after therapy is low or decreased compared to glycodelin level before said therapy.

8. Glycodelin for use in modulating the immune system in a subject with lung transplantation,
comprising treatment of the lung with glycodelin before, during and/or after said lung transplantation.

9. A method for the diagnosis, prognosis and/or treatment monitoring of a lung disease, comprising the step of
determining the presence of glycodelin in serum, plasma and/or tissue, such as tumor tissue,
wherein the lung disease is selected from thoracic malignancies, mesothelioma, lung metastasis from distant malignant diseases and benign thoracic diseases,
wherein, preferably, a thoracic malignancy is lung cancer which is more preferably selected from non-small cell lung cancer (NSCLC) (such as adenocarcinoma, squamous cell carcinoma, large-cell lung carcinoma), small cell lung cancer (SCLC), lung carcinoid.

10. The method according to claim 9, wherein determining the presence of glycodelin comprises
- determining presence / expression of glycodelin mRNA,
preferably via quantitative detection of the mRNA,
and/or
- determining presence / expression of glycodelin
preferably via immune assay(s) or immunohistochemistry, such as by using specific anti-glycodelin antibodies.

11. The method according to claim 10, wherein glycodelin is not detectable in normal lung parenchyma,
and glycodelin is detectable in bronchial epithelium of the lung and in tumor tissue, preferably glycodelin is increased in bronchial epithelium of the lung and in tumor tissue compared to normal lung parenchyma or glycodelin is above a threshold (value) or cut-off (value).

12. The method according to any of claims 9 to 11, wherein diagnosis of a lung disease comprises the early diagnosis of the lung disease,
and/or wherein prognosis of a lung disease comprises assessment of progression-free survival, disease-free survival and overall survival,
and/or wherein treatment monitoring comprises determining the presence of glycodelin over time,
such as before, during and/or after therapy (such as surgery, chemo-, immune- and/or radiotherapy; or combined modality treatment) and/or during follow-up after therapy.

13. The method according to claim 12, wherein a therapy is found successful, if the glycodelin level during and/or after therapy is low or decreased compared to glycodelin level before said therapy.

14. A method for modulating the immune system in a subject with lung transplantation, comprising the step of
treatment of the lung with glycodelin before, during and/or after said lung transplantation.


SEQUENCE LISTING

<110>  Thoraxklinik-Heidelberg gGmbH

<120>  Glycodelin as diagnostic and prognostic marker and for monitoring
       treatment of lung diseases

<130>  T31425EP

<160>  2

<170>  PatentIn version 3.5

<210>  1
<211>  5022
<212>  DNA
<213>  Homo sapiens

<400>  1
cagcatccct ctggctccag agctcagagc cacccacagc cgcagccatg ctgtgcctcc      60

tgctcaccct gggcgtggcc ctggtctgtg gtgtcccggc catggacatc ccccagacca     120

agcaggacct ggagctccca aaggtttgag gctgggggag cgggcacttt actgtgggag     180

gcctggggcg ggtgggagct gcgggcaggc aggaagccca ggatctcaga aacctacagg     240

aagcacagaa tggacgccat gacgtcagga agccctcagc cctgctctcc atctttaggg     300

tggcctctct ggtttcccag catcctaggt gactcattat ttggactttg gaacactcct     360

gagttagcac acactggtca ttttaagtac aggaaatttc atagcccagg atctggtaga     420

tagcagacaa ccatccaatg ctcactgtac ccatcccagt tagactcagc cccgtctgca     480

ccgggtgcaa cgagagccat ggtggggtgg gaccgccgtg cagcccaagg ccccctcagt     540

tggcagggac ctggcactcc atggccatgg cgaccaacaa catctccctc atggcgacac     600

tgaaggcccc tctgagggtc cacatcacct cactgttgcc cacccccgag gacaacctgg     660

agatcgttct gcacagatgg tgggtttctc atcattgaga cgggctgggc gggggctcag     720

tctcccccct caggggtcca ggactgggtg ggttgggcgg agctggactt agccccaggc     780

attttctgac agccaggggc ttcactgtgg cccttccatg agggtggggt ggaaaaccag     840

ggctccagac gttccctgtc cccttggatc ccctgcccca ggctctgggc caacagccaa     900

ccacacagtg cagccccagg tcagactgag gagaaggtct gggcggctgc gggctgcggt     960

gctccttgga cccggggaag ttcccgtggt gacctgattt taggagtgac agtgaaggca    1020

actccaattc aagtggccac tcatcctatt gtcaccacct ttcagggaga acaacagctg    1080

tgttgagaag aaggtccttg gagagaagac tgagaatcca aagaagttca agatcaactg    1140

tgagtgtccc caggccccaa gggctggctc agtgctggca tgctagccac gctctcccag    1200

aggcggctct gctggggcat gagggagtgg ggcctggcct gtccccactc tctctgcttc    1260

agggagtcag agtgtttact ccggtcaacc tgatgctgac cccagaggca tcttttacct    1320

ggagggcagg ggaagcacta attcttggca tgacatgact ggatgtgggt ctgcactgtg 1380
cccaggcaca ggggacaggt gctttgttgc actgttcact ctggcctcac aaaaggccag 1440
ggaggctgca ggcgagcagg tgggcaggtg ggcaggtggg taggtgggta ggtggatatg 1500
tatacaggtg ggcaggaggg taggtgaaca ggtgggtagg tgggcaggtg gctaggtgag 1560
taagtggtta ggtgaacagg tgggcaggtg agcaggtggt taggtgaaca ggtgggcagg 1620
tgggtaggtg ggtaggtata caggtggaca ggtgggtagg tggacaggtg ggcaggtgag 1680
taggtgaaca ggtgggtggg tgaacaggtg gccaggtgaa caagttggta ggtgggcagg 1740
tgggtaggtg aacaggtggg caggtgggca ggtgggcagg tgggcaggtg ggcaagtggc 1800
tgctgttccc gtgggcctgg ctgcctcctg cgcactctgg ggctgcagct ctggtcttag 1860
gctgagctcc caggcctctc tgggggaaga gagaggggct tacagcatgt ccttggtcca 1920
ctgaattctt cctaacaatt tgcaacattt tgttctattt tgttaattat tattttttta 1980
aaaagacaga ggtggtcagg gtctggggcc tcttatcccc tcatgggcac attttcccag 2040
caaatacagt ttgcttctca tgcttgggac ttgcctcagg cctttctgac cctgcttgcc 2100
ctccccagaa tcgagccact ctccaaggtc catttcttct ccctcttccc gcccctgtgc 2160
cctgttcctg tgccatctcc cgccatcctc acccgtacgt gacttctcag ttggagtctc 2220
tccaggtcac agcctccctg cctgccgtgt ctgcctctcc acggcacacc tggcctctcc 2280
ccctcagccg gggctccatg gccctccaca ttgcctctcc tcccctttcc tccctggctt 2340
ccctgatcat ggtccacagc aggggccacg tcccatggtg tcagtggatg aggaagccac 2400
ttagtgtggt gggatgtcca cacacctgca caggactctg ctgagacgga ggcttcatct 2460
tccttttggt tcttctcttc tttccccaga tacggtggcg aacgaggcca cgctgctcga 2520
tactgactac gacaatttcc tgtttctctg cctacaggac accaccaccc ccatccagag 2580
catgatgtgc cagtacctgg gtgggtctca cagcacatga gctcaacgtg ggtgagaggc 2640
agcagctacg tccgtggctg ggaaccctgg ggagctgaca actggcttcc tgtccacctc 2700
ggtgcctgtg ggctgacttt ttttttcttg gtttttttta tttgtttgtt gtttgctttg 2760
tttttttgag aaagggtctc attctgtcac tcaggctgga gtgtagtgac gtgatctcgg 2820
ctcactgcaa cttctgcctc ccagattcaa gcgattctcg tgcctcagcc tccagagtag 2880
ctgggattac aggcgcacgc caccatgccc agctaatttt tgtatttttt ggtagagacg 2940
gggtttcacc atgttggcca ggctggtctc caactcctgg atcaagtgat ccacccgcct 3000
tggcctccca aagggctggg attacaggcg tgagccacca cgcccggcca ggctgagttt 3060
ttctccagcg gttcatcgag tcctctgaca aagcaaggag ctgatatagg gccagtggga 3120
cggtcgccag tcaaggggct gggcttggtg gatagattaa tactcactgg gcgtccagtc 3180
aaaacgccct gaaacctatg atgctgtcaa ccaaacgaag gccaggaata ccaaaatagc 3240

cacataggca cagcccttcc ccatgtttct gagcacagtg tttcctctgg ggtcacacag 3300

gtgtcttctt gatcagcctc agccatgctt ggtgagagcc gggcactggg agagccaggc 3360

actgtgctct cctgtgacgc tgtagacacc atcctaagct gtgcagaccc cagcgctgcc 3420

cagagcggag cagagggggc cgggccaagg agtgggagct ggggtcaggg aacctggagg 3480

tgcagtggac agagccccgg agaccgccct agggacctac tccagaccaa actctgccag 3540

acctcggagc actggggcct ccttctctgc cctccctcct caggcaaggc ctctggagct 3600

ccccagctct catggaagcc ccaggggccc aggactgacc cagcctcttc cacagccaga 3660

gtcctggtgg aggacgatga gatcatgcag ggattcatca gggctttcag gcccctgccc 3720

aggcacctat ggtacttgct ggacttgaaa cagatggaag gtgagctctg cctaggacac 3780

gcccagcctc agctggagga gaagctgcct ctttcttagc ccgagccccc tgctggctct 3840

gcaggactca ggtcactcct ttttggcccc tcccctgttc tcccctggcc ttctggggtg 3900

cagagccacc ctgaggtggg gtcctgccct ctcccaccat cctttcatcc cttctctagc 3960

cctggggctg ctgtgtcccc agctgtctct tctctcgctg acacctccac tgtcccatct 4020

cctcccacag agccgtgccg tttctaggtg agctcctgcc tggtcctgcc tcctgggtaa 4080

tgtatcagcc tcgcccactg tctgcggctg cctctctggg cccctgggac agaccctact 4140

gtgtccagtt cagggctgac cctacaggaa tgaactgggg tctggtcttg tgattccaga 4200

aagccaggct gctgacgtcc ccattcacga gcccagcctg tgtcttgcag ccattgtatt 4260

agtcacgggc ttgtgcccta tagtcagacc tcatgctttc ttttggggtt aggggtgttg 4320

gttggaaatg gtgggggcta taggaggagg aaggaggatg gttacatgga agggcatgag 4380

aagctgggga cctgcaggtc tcggtcccac gttctttttt ttttttcttt ttttaagatg 4440

gagtctcgct ctgtcaccag gctggagtgc agtggcacaa tctcagctca ctgcaacctc 4500

gacctcctgg gttcaagcga ttctcctgcc tcaacccccc gagttgctgg aaccacaggc 4560

gtgtgccacc atgcccagct aatttttgta tttttaatag aaacggggtt tcaccatgtt 4620

ggccacgatg gtctcaatct cttgacctca tgatcccccc gctttagcct cccaaagtgc 4680

tgggatttca gtgccacatt cttaaggggg tgtcctcaag cccaccacat ccttccaggg 4740

ctcccccgaa acaccctgct cttcctccct ctacttaagt gacctgtaaa cccaacagct 4800

cacctccgcc tccaggaaga ccagactccc acccttccac acctccagag cagtgggact 4860

tcctcctgcc ctttcaaaga ataaccacag ctcagaagac gatgacgtgg tcatctgtgt 4920

cgccatcccc ttcctgctgc acacctgcac cacggccatg gggaggctgc tccctggggg 4980

cagagtctct ggcagaggtt attaataaac ccttggagca tg 5022

<210> 2

<211>  180
<212>  PRT
<213>  Homo sapiens

<400>  2

Met Leu Cys Leu Leu Leu Thr Leu Gly Val Ala Leu Val Cys Gly Val
1               5                   10                  15

Pro Ala Met Asp Ile Pro Gln Thr Lys Gln Asp Leu Glu Leu Pro Lys
            20                  25                  30

Leu Ala Gly Thr Trp His Ser Met Ala Met Ala Thr Asn Asn Ile Ser
        35                  40                  45

Leu Met Ala Thr Leu Lys Ala Pro Leu Arg Val His Ile Thr Ser Leu
    50                  55                  60

Leu Pro Thr Pro Glu Asp Asn Leu Glu Ile Val Leu His Arg Trp Glu
65                  70                  75                  80

Asn Asn Ser Cys Val Glu Lys Lys Val Leu Gly Glu Lys Thr Glu Asn
                85                  90                  95

Pro Lys Lys Phe Lys Ile Asn Tyr Thr Val Ala Asn Glu Ala Thr Leu
            100                 105                 110

Leu Asp Thr Asp Tyr Asp Asn Phe Leu Phe Leu Cys Leu Gln Asp Thr
        115                 120                 125

Thr Thr Pro Ile Gln Ser Met Met Cys Gln Tyr Leu Ala Arg Val Leu
    130                 135                 140

Val Glu Asp Asp Glu Ile Met Gln Gly Phe Ile Arg Ala Phe Arg Pro
145                 150                 155                 160

Leu Pro Arg His Leu Trp Tyr Leu Leu Asp Leu Lys Gln Met Glu Glu
                165                 170                 175

Pro Cys Arg Phe
            180